# EUROPEAN PATENT SPECIFICATION

(11) **EP 3 405 177 B1**
(45) Date of publication and mention of the grant of the patent: **11.03.2026**
(21) Application number: 17700820.8
(22) Date of filing: 18.01.2017
(51) Int. Cl.: A61K 31/519, A61K 45/06, A61P 3/04, A61P 17/00, A61P 17/02, A61P 19/10, A61P 29/00, A61P 37/00, A61P 37/02, A61P 43/00, A61K 9/20, A61K 9/48, A61P 35/00, A61P 35/02, A61P 35/04

(54) **FORMULATIONS/COMPOSITIONS COMPRISING A BTK INHIBITOR**
FORMULIERUNGEN/ZUSAMMENSETZUNGEN MIT EINEM BTK-INHIBITOREN
FORMULATIONS/COMPOSITIONS COMPRENANT UN INHIBITEUR DE BTK

(30) Priority: 19.01.2016 IN 201621001986
(43) Date of publication of application: 28.11.2018
(73) Proprietor: Janssen Pharmaceutica NV, 2340 Beerse (BE)
(72) Inventor: GUPTA, Manish, Kumar, 2340 Beerse (BE); KULKARNI, Parikshit, Rameshrao, Pandharpur Maharashtra 413304 (IN); KRISHNAN NAIR, Binuraj, Dombivali East Maharashtra 421204 (IN)
(74) Representative: Purewal, Savroop
(86) International application number: PCT/EP2017/050962
(87) International publication number: WO 2017/125423

(56) References cited:
- WO-A1-2014/004707
- WO-A1-2016/022942
- WO-A1-2016/141068
- CN-A- 104 523 695
- US-A1- 2014 336 203

## Description

### Field of the invention

The present invention relates to formulations of a Bruton's tyrosine kinase (BTK) inhibitor, particularly ibrutinib. It also relates to processes for preparing such formulations/compositions comprising a BTK inhibitor as well as methods of using such formulations/compositions in the treatment of diseases or conditions that would benefit from inhibition of BTK activity.

### Background of the Invention

Ibrutinib is an organic small molecule having IUPAC name 1-[(3*R*)-3-[4-amino-3-(4-phenoxyphenyl)pyrazolo[3,4-d]pyrimidin-1-yl]piperidin-1-yl]prop-2-en-1-one. It is described in a number of published documents, including international patent application WO 2008/039218 (Example 1b), and is described as an irreversible inhibitor of Btk. Various ibrutinib formulations have been disclosed, for instance in US patent application US 2014/3366203 and PCT patent application WO 2014/004707.

Btk plays an essential role in the B-cell signaling pathway linking cell surface B-cell receptor stimulation to downstream intracellular responses. Btk is a key regulator of B-call development, activation, signaling, and survival (Kurosaki, Curr Op Imm, 2000, 276-281; Schaeffer and Schwartzberg, Curr Op Imm 2000, 282-288). In addition, Btk plays a role in a number of other hematopoetic cell signaling pathways, e.g. Toll like receptor (TLR) and cytokine receptor-mediated TNF-α production in macrophages, IgE receptor (FcepsilonRI) signaling in Mast cells, inhibition of Fas/APO-1 apoptotic signaling in B-lineage lymphoid cells, and collagen-stimulated platelet aggregation. See e.g., C. A. Jeffries, et al., (2003), Journal of Biological Chemistry 278:26258-26264; N. J. Horwood, et al., (2003), The Journal of Experimental Medicine 197:1603-1611; Iwaki et al. (2005), Journal of Biological Chemistry 280(48):40261-40270; Vassilev et al. (1999), Journal of Biological Chemistry 274(3):1646-1656, and Quek et al (1998), Current Biology 8(20):1137-1140.

Ibrutinib therefore plays a role in targeting B-cell malignancies. Ibrutinib blocks signals that stimulate malignant B cells to grow and divide uncontrollably. It is therefore being studied in clinical trials for various hematological malignancies such as chronic lymphocytic leukemia, mantle cell lymphoma, diffuse large B-cell lymphoma, Waldenstrom's macroglobulinemia and multiple myeloma. It has also received regulatory approval in some counties for certain conditions. For example it was approved by the US FDA in November 2013 for the treatment of mantle cell lymphoma, in February 2014 for the treatment of chronic lymphocytic leukemia and in January 2015 for the treatment of Waldenstom's macroglobulinemia.

Alternative formulations of ibrutinib are required and/or desired.

### Summary of the Invention

In one aspect, there is now provided a pharmaceutical composition comprising ibrutinib, wherein ibrutinib is a compound with the structure of Compound 1, 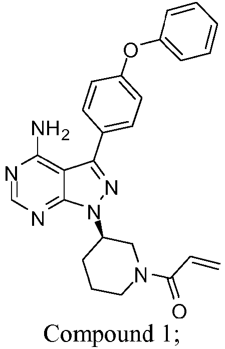 and wherein the pharmaceutical composition comprises i) at least 50% w/w to 80% w/w of ibrutinib, and ii) excipients comprising about 10-30% w/w of filler, that is microcrystalline cellulose (e.g. silicified microcrystalline cellulose) of the total weight of the pharmaceutical composition as defined by the claims.

The pharmaceutical composition further comprising excipients comprising 5-20% w/w of disintegrant (e.g. crospovidone) of the total weight of the pharmaceutical composition.

In an aspect is a pharmaceutical composition wherein the excipients comprise a filler containing a water-soluble filler such as one or more polyol (e.g. one or more sugars such as mannitol, lactose and the like). Such water-soluble filler (e.g. polyol(s)) is, in an embodiment, in addition to another filler (for example microcrystalline cellulose).

In an aspect is a pharmaceutical composition wherein the excipients comprise a filler containing microcrystalline cellulose and, optionally, one or more (e.g. one) further ingredient such as a water-soluble filler such as one or more polyols (e.g. one or more sugars such as mannitol, lactose and the like).

In another aspect is a pharmaceutical composition wherein the excipients do not comprise a filler that is mannitol (or, for example the microcrystalline cellulose is the only filler as a component of the pharmaceutical composition). In another aspect is a pharmaceutical composition wherein the excipients comprise a filler that only contains microcrystalline cellulose.

The microcrystalline cellulose that may be employed in the process of the invention may be from any suitable source, for example it may be silicified microcrystalline cellulose (SMCC) such as SMCC HD90 (e.g. PROSOLV SMCC^{®}, which may be available through JRS Pharma). SMCC (e.g. SMCC HD90) may produce advantages associated with - high compactibility - high intrinsic flow - enhanced lubrication efficiency - improved blending properties.

According to the invention the pharmaceutical composition comprises about 50% w/w to about 80% w/w of ibrutinib. In another embodiment is a pharmaceutical composition, wherein the pharmaceutical composition comprises about 60% w/w to about 80% w/w of ibrutinib. In another embodiment is a pharmaceutical composition wherein the pharmaceutical composition comprises about 60% w/w to about 70% w/w of ibrutinib. In another embodiment is a pharmaceutical composition wherein the pharmaceutical composition comprises about 60% or about 70% w/w of ibrutinib.

According to the invention the pharmaceutical composition comprises intragranular and extragranular ingredients.

In another aspect is a pharmaceutical composition wherein ibrutinib and the filler (e.g. microcrystalline cellulose) are intragranular ingredients.

In another aspect is a pharmaceutical composition wherein the pharmaceutical composition comprises about 10% w/w to about 25% w/w of the filler (e.g. microcrystalline cellulose). In another embodiment is a pharmaceutical composition wherein the pharmaceutical composition comprises about 22-23% w/w of the filler (being microcrystalline cellulose). In another embodiment is a pharmaceutical composition wherein the pharmaceutical composition comprises about 12% w/w of the filler (being microcrystalline cellulose).

In another aspect is a pharmaceutical composition wherein crospovidone is an intragranular and extragranular ingredient. In another embodiment is a pharmaceutical composition wherein the pharmaceutical composition comprises about 8% w/w to about 12% w/w of crospovidone. In another embodiment is a pharmaceutical composition wherein the pharmaceutical composition comprises about 10% w/w of crospovidone.

In another aspect is a pharmaceutical composition wherein the pharmaceutical composition comprises about 60% w/w of ibrutinib, about 22-23% w/w of filler (being microcrystalline cellulose), and about 10% w/w of crospovidone.

In another aspect is a pharmaceutical composition wherein the pharmaceutical composition comprises about 70% w/w of ibrutinib, about 12% w/w of filler (being microcrystalline cellulose), and about 10% w/w of crospovidone.

According to the invention is a pharmaceutical composition wherein the pharmaceutical composition is prepared using a dry granulation method (e.g. in an embodiment, a roller compaction process; in another embodiment, a high shear granulation). In an embodiment, the dry granulation method is a roller compaction process, and, in such an embodiment, when microcrystalline cellulose is present (e.g. as an excipient that is a filler), then in an aspect, SMCC is employed (e.g. SMCC HD90). Such use of a roller compaction process (e.g. in an aspect, in the aspect where SMCC, such as SMCC HD90 is employed) may have advantages associated with compression/compaction, providing tablets with adequate hardness, and providing enhanced lubrication efficiency, for instance no sticking/picking observed during tableting.

In another aspect is a pharmaceutical composition further comprising at least one additional pharmaceutically acceptable excipient.

In yet another aspect is a high-load solid tablet formulation comprising a pharmaceutical composition as described herein, and one or more additional pharmaceutically acceptable excipients. In another embodiment is a high-load solid tablet formulation, wherein the one or more additional excipients are present in an amount from about 7% w/w to about 13% w/w. In another embodiment is a high-load solid tablet formulation, wherein the one or more additional excipients are selected from the group consisting of binders, lubricants, glidants, and surfactants.

In another embodiment is a high-load solid tablet formulation, wherein at least one additional excipient is a surfactant. In another embodiment is a high-load solid tablet formulation, wherein at least one additional excipient is present that is a surfactant is sodium lauryl sulfate. In another embodiment is a high-load solid tablet formulation, wherein (when at least additional excipient is present that is the surfactant sodium lauryl sulfate) the sodium lauryl sulfate is present in an amount from about 0 to about 10% w/w, about 4% w/w to about 8% w/w, or about 4% w/w to about 6% w/w (in a further embodiment, the sodium lauryl sulfate is present in an amount of about 4% w/w or about 5% w/w; and in yet a further embodiment, the sodium lauryl sulfate is present in an amount of about 0.5% w/w to about 4%).

In another embodiment is a high-load solid tablet formulation, wherein at least one additional excipient is a glidant. In another embodiment is a high-load solid tablet formulation, wherein at least one additional excipient is present that is a glidant that is silica (colloidal silicon dioxide). In another embodiment is a high-load solid tablet formulation, wherein (when at least additional excipient is present that is the glidant silica) the silica (colloidal silicon dioxide) is present in an amount from about 0 to about 5% w/w, 0.1% w/w to about 1.5% w/w, about 0.4% w/w to about 0.8% w/w, or about 0.5% w/w.

In another embodiment is a high-load solid tablet formulation, wherein at least one additional excipient is a lubricant. In another embodiment is a high-load solid tablet formulation, wherein at least one additional excipient is present that is a lubricant that is magnesium stearate. In another embodiment is a high-load solid tablet formulation, wherein (when at least additional excipient is present that is the lubricant magnesium stearate) the magnesium stearate is present in an amount from about 0.01% w/w to about 5% w/w, 0.01% w/w to about 2% w/w, 0.1% w/w to about 0.7% w/w, or about 0.3% w/w to about 0.5% w/w.

In another aspect is a high-load solid tablet formulation, wherein a binder (e.g. polyvinylpyrrolidone) is not present in the formulation (as an excipient).

In another aspect is a high-load solid tablet formulation, wherein the total weight of a tablet is about 800 mg. In another embodiment is a high-load solid tablet wherein ibrutinib is in an amount of about 560 mg. In another embodiment is a high-load solid tablet wherein ibrutinib is in micronized form.

In another aspect is a high-load solid tablet formulation, wherein the formulation is used for once a day dosing. In another embodiment is a high-load solid tablet wherein the formulation is in an oral dosage form. Any references in the description to methods of treatment, diagnosis or surgery refer to the compounds, pharmaceutical compositions and medicaments of the present invention for use in a method for treatment, diagnosis or surgery of the human or animal body by therapy.

In another aspect is a method of treating a disease in a patient in need of such treatment, comprising administering to the patient a therapeutically effective amount of a pharmaceutical composition or formulation described herein.

In another aspect is a method of treating an autoimmune disease or condition in a patient in need of such treatment, comprising administering to the patient a therapeutically effective amount of a pharmaceutical composition or formulation described herein. In some embodiments, the autoimmune disease is rheumatoid arthritis or lupus.

In another aspect is a method of treating a heteroimmune disease or condition in a patient in need of such treatment, comprising administering to the patient a therapeutically effective amount of a pharmaceutical composition or formulation described herein.

In another aspect is a method of treating cancer in a patient in need of such treatment, comprising administering to the patient a therapeutically effective amount of a pharmaceutical composition or formulation described herein. In some embodiments, the cancer is a B-cell proliferative disorder. In some embodiments, the B-cell proliferative disorder is diffuse large B cell lymphoma, follicular lymphoma or chronic lymphocytic leukemia. In some embodiments, the cancer is a B cell malignancy. In some embodiments, the cancer is a B cell malignancy selected from chronic lymphocytic leukemia (CLL)/ small lymphocytic lymphoma (SLL), mantle cell lymphoma (MCL), diffuse large B Cell lymphoma (DLBCL), and multiple myeloma. In some embodiments, the cancer is a lymphoma, leukemia or a solid tumor. In some embodiments, the cancer is diffuse large B cell lymphoma, follicular lymphoma, chronic lymphocytic lymphoma, chronic lymphocytic leukemia, B-cell prolymphocytic leukemia, lymphoplasmacytic lymphoma/Waldenström macroglobulinemia, splenic marginal zone lymphoma, plasma cell myeloma, plasmacytoma, extranodal marginal zone B cell lymphoma, nodal marginal zone B cell lymphoma, mantle cell lymphoma, mediastinal (thymic) large B cell lymphoma, intravascular large B cell lymphoma, primary effusion lymphoma, burkitt lymphoma/leukemia, or lymphomatoid granulomatosis.

In another aspect is a method of treating mastocytosis in a patient in need of such treatment, comprising administering to the patient a therapeutically effective amount of a pharmaceutical composition or formulation described herein.

In another aspect is a method of treating osteoporosis or bone resorption disorders in a patient in need of such treatment, comprising administering to the patient a therapeutically effective amount of a pharmaceutical composition or formulation described herein.

In another aspect is a method of treating an inflammatory disease or condition in a patient in need of such treatment, comprising administering to the patient a therapeutically effective amount of a pharmaceutical composition or formulation described herein.

In another aspect is a method of treating lupus in a patient in need of such treatment, comprising administering to the patient a therapeutically effective amount of a pharmaceutical composition or formulation described herein.

In another aspect is a process for preparing a pharmaceutical composition (e.g. as described herein) or a tablet formulation (e.g. as described herein) comprising ibrutinib, the process comprising preparing dry granules comprising ibrutinib and at least one excipient as defined by the claims, by a dry granulation method (e.g. a roller granulation method).

In an embodiment, the process may be described with reference to the following steps: (i) blend sieved sodium lauryl sulfate, silicified microcrystalline cellulose and Crospovidone in a blender; (ii) blend with sieved one half of the amount of micronized Ibrutinib; (iii) blend with sieved, the remaining half of micronized Ibrutinib; (iv) the blended mass is lubricated with sieved magnesium stearate in a blender; (v) compact the blend (intraganular) through roller compactor; (vi) pass compacted material through mill; (vii) blend milled material with the second portion of sieved Crospovidone and sodium lauryl sulfate along with colloidal silicon dioxide in a blender; (viii) the blended granulation is lubricated with the second portion of sieved magnesium stearate in a blender; (ix) final blend is compressed into tablets using rotary compression machine fitted with suitable tooling; (x) tablets are film coated using coating machine; and (xi) package tablets using conventional procedure.

In another aspect is a high-load solid tablet formulation comprising ibrutinib, wherein ibrutinib is a compound with the structure of Compound 1, 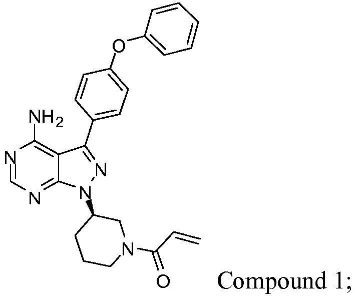 and the tablet comprises about 560 mg of ibrutinib.

In another embodiment is a high-load solid tablet formulation, wherein ibrutinib is in micronized form. In another embodiment, ibrutinib is in spray-dried form. In another embodiment, the particle size is about or less than 30 micron. In one embodiment, ibrutinib is in micronized form and the particle size is about 1-30 micron. In another embodiment, the particle size is about or less than 10 micron. In another embodiment, the particle size is <1 micron. In another embodiment is a high-load solid tablet formulation, wherein the tablet is used for once a day oral dosing.

In another aspect, provided herein are methods for treating a patient by administering Compound 1. In some embodiments, provided herein is a method of inhibiting the activity of tyrsoine kinase(s), such as Btk, or of treating a disease, disorder, or condition, which would benefit from inhibition of tyrosine kinase(s), such as Btk, in a mammal, which includes administering to the mammal a therapeutically effective amount of Compound 1, or pharmaceutically acceptable salt, pharmaceutically active metabolite, pharmaceutically acceptable prodrug, or pharmaceutically acceptable solvate.

In another aspect, provided herein is the use of Compound 1 for inhibiting Bruton's tyrosine kinase (Btk) activity or for the treatment of a disease, disorder, or condition, which would benefit from inhibition of Bruton's tyrosine kinase (Btk) activity.

In some embodiments, a pharmaceutical composition comprising crystalline Compound 1 is administered to a human. In some embodiments, a pharmaceutical composition comprising amorphous Compound 1 is administered to a human.

In some embodiments, a pharmaceutical composition comprising crystalline Compound 1 is orally administered. In some embodiments, a pharmaceutical composition comprising amorphous Compound 1 is orally administered.

In some embodiments, a pharmaceutical composition comprising crystalline Compound 1 is used for the formulation of a medicament for the inhibition of tyrosine kinase activity. In some other embodiments, a pharmaceutical composition comprising crystalline Compound 1 is used for the formulation of a medicament for the inhibition of Bruton's tyrosine kinase (Btk) activity. In some embodiments, a pharmaceutical composition comprising amorphous Compound 1 is used for the formulation of a medicament for the inhibition of tyrosine kinase activity. In some other embodiments, a pharmaceutical composition comprising amorphous Compound 1 is used for the formulation of a medicament for the inhibition of Bruton's tyrosine kinase (Btk) activity.

In some embodiments, in any of the embodiments disclosed herein (including compositions, methods, uses, formulations, combination therapy, etc.), Compound 1, or a pharmaceutically acceptable salt or solvate thereof, is optically pure (i.e. greater than 99% chiral purity by HPLC). In some embodiments, in any of the embodiments disclosed herein (including compositions, methods, uses, formulations, combination therapy, etc.), Compound 1, or a pharmaceutically acceptable salt or solvate thereof, is replaced with: a) Compound 1, or a pharmaceutically acceptable salt or solvate thereof, of lower chiral purity; b) 1-((S)-3-(4-amino-3-(4-phenoxyphenyl)-1H-pyrazolo[3,4-d]pyrimidin-1-yl)piperidin-1-yl)prop-2-en-1-one, or a pharmaceutically acceptable salt or solvate thereof of any optical purity; or c) racemic 1-(3-(4-amino-3-(4-phenoxyphenyl)-1H-pyrazolo[3,4-d]pyrimidin-1-yl)piperidin-1-yl)prop-2-en-1-one, or a pharmaceutically acceptable salt or solvate thereof.

In any of the embodiments disclosed herein (including compositions, methods, uses, formulations, combination therapy, etc.), amorphous Compound 1 is used. In any of the embodiments disclosed herein (including compositions, methods, uses, formulations, combination therapy, etc.), crystalline Compound 1 is used.

In some embodiments, in any of the embodiments disclosed herein (including compositions, methods, uses, formulations, combination therapy, etc.), Compound 1, or a pharmaceutically acceptable salt thereof, is replaced with an active metabolite of Compound 1. In some embodiments, the active metabolite is in a crystalline form. In some embodiments, the active metabolite is in an amorphous phase. In further embodiments the metabolite is isolated. In some embodiments, in any of the embodiments disclosed herein (including compositions, methods, uses, formulations, combination therapy, etc.), Compound 1, or a pharmaceutically acceptable salt thereof, is replaced with a prodrug of Compound 1, or a deuterated analog of Compound 1, or a pharmaceutically acceptable salt thereof.

Other objects, features and advantages of the methods and compositions described herein will become apparent from the following detailed description.

The section headings used herein are for organizational purposes only and are not to be construed as limiting the subject matter described.

### BRIEF DESCRIPTION OF THE FIGURES

All the four Figures show a comparison between "Treatment A" (as defined hereinafter) and "Treatment C" (also defined hereinafter)
FIG 1 shows linear-linear mean plasma ibrutinib concentration vs time profiles from 0 to 12 hours
FIG 2 shows logarithmic-linear mean plasma ibrutinib concentration vs time profiles from 0 to 12 hours
FIG 3 shows linear-linear mean plasma ibrutinib concentration vs time profiles from 0 to 48 hours
FIG 4 shows logarithmic-linear mean plasma ibrutinib concentration vs time profiles from 0 to 48 hours

### DETAILED DESCRIPTION OF THE INVENTION

The diverse roles played by Btk signaling in various hematopoietic cell functions, e.g., B-cell receptor activation, suggests that small molecule Btk inhibitors, such as Compound 1, are useful for reducing the risk of or treating a variety of diseases affected by or affecting many cell types of the hematopoietic lineage including, e.g., autoimmune diseases, heteroimmune conditions or diseases, inflammatory diseases, cancer (e.g., B-cell proliferative disorders), and thromboembolic disorders. Further, irreversible Btk inhibitor compounds, such as Compound 1, can be used to inhibit a small subset of other tyrosine kinases that share homology with Btk by having a cysteine residue (including a Cys 481 residue) that can form a covalent bond with the irreversible inhibitor.

In some embodiments, the compositions or tablet formulations comprising Compound 1 as defined by the claims, can be used in the treatment of an autoimmune disease in a mammal, which includes, but is not limited to, rheumatoid arthritis, psoriatic arthritis, osteoarthritis, Still's disease, juvenile arthritis, lupus, diabetes, myasthenia gravis, Hashimoto's thyroiditis, Ord's thyroiditis, Graves' disease Sjögren's syndrome, multiple sclerosis, Guillain-Barré syndrome, acute disseminated encephalomyelitis, Addison's disease, opsoclonus-myoclonus syndrome, ankylosing spondylitisis, antiphospholipid antibody syndrome, aplastic anemia, autoimmune hepatitis, coeliac disease, Goodpasture's syndrome, idiopathic thrombocytopenic purpura, optic neuritis, scleroderma, primary biliary cirrhosis, Reiter's syndrome, Takayasu's arteritis, temporal arteritis, warm autoimmune hemolytic anemia, Wegener's granulomatosis, psoriasis, alopecia universalis, Behçet's disease, chronic fatigue, dysautonomia, endometriosis, interstitial cystitis, neuromyotonia, scleroderma, and vulvodynia.

In some embodiments, the compositions or tablet formulations comprising Compound 1, as defined by the claims, can be used in the treatment of a heteroimmune disease or condition in a mammal, which include, but are not limited to graft versus host disease, transplantation, transfusion, anaphylaxis, allergies (e.g., allergies to plant pollens, latex, drugs, foods, insect poisons, animal hair, animal dander, dust mites, or cockroach calyx), type I hypersensitivity, allergic conjunctivitis, allergic rhinitis, and atopic dermatitis.

In some embodiments, the compositions or tablet formulations comprising Compound 1, as defined by the claims, can be used in the treatment of an inflammatory disease in a mammal, which includes, but is not limited to asthma, inflammatory bowel disease, appendicitis, blepharitis, bronchiolitis, bronchitis, bursitis, cervicitis, cholangitis, cholecystitis, colitis, conjunctivitis, cystitis, dacryoadenitis, dermatitis, dermatomyositis, encephalitis, endocarditis, endometritis, enteritis, enterocolitis, epicondylitis, epididymitis, fasciitis, fibrositis, gastritis, gastroenteritis, hepatitis, hidradenitis suppurativa, laryngitis, mastitis, meningitis, myelitis myocarditis, myositis, nephritis, oophoritis, orchitis, osteitis, otitis, pancreatitis, parotitis, pericarditis, peritonitis, pharyngitis, pleuritis, phlebitis, pneumonitis, pneumonia, proctitis, prostatitis, pyelonephritis, rhinitis, salpingitis, sinusitis, stomatitis, synovitis, tendonitis, tonsillitis, uveitis, vaginitis, vasculitis, and vulvitis. In some embodiments, the inflammatory disease is asthma, appendicitis, blepharitis, bronchiolitis, bronchitis, bursitis, cervicitis, cholangitis, cholecystitis, colitis, conjunctivitis, cystitis, dacryoadenitis, dermatitis, dermatomyositis, encephalitis, endocarditis, endometritis, enteritis, enterocolitis, epicondylitis, epididymitis, fasciitis, fibrositis, gastritis, gastroenteritis, hepatitis, hidradenitis suppurativa, laryngitis, mastitis, meningitis, myelitis myocarditis, myositis, nephritis, oophoritis, orchitis, osteitis, otitis, pancreatitis, parotitis, pericarditis, peritonitis, pharyngitis, pleuritis, phlebitis, pneumonitis, pneumonia, proctitis, prostatitis, pyelonephritis, rhinitis, salpingitis, sinusitis, stomatitis, synovitis, tendonitis, tonsillitis, uveitis, vaginitis, vasculitis, or vulvitis. In some embodiments, the autoimmune disease is inflammatory bowel disease, arthritis, lupus, rheumatoid arthritis, psoriatic arthritis, osteoarthritis, Still's disease, juvenile arthritis, diabetes, myasthenia gravis, Hashimoto's thyroiditis, Ord's thyroiditis, Graves' disease Sjögren's syndrome, multiple sclerosis, Guillain-Barré syndrome, acute disseminated encephalomyelitis, Addison's disease, opsoclonus-myoclonus syndrome, ankylosing spondylitisis, antiphospholipid antibody syndrome, aplastic anemia, autoimmune hepatitis, coeliac disease, Goodpasture's syndrome, idiopathic thrombocytopenic purpura, optic neuritis, scleroderma, primary biliary cirrhosis, Reiter's syndrome, Takayasu's arteritis, temporal arteritis, warm autoimmune hemolytic anemia, Wegener's granulomatosis, psoriasis, alopecia universalis, Behçet's disease, chronic fatigue, dysautonomia, endometriosis, interstitial cystitis, neuromyotonia, scleroderma, or vulvodynia.

In yet other embodiments, the methods described herein can be used to treat a cancer, e.g., B-cell proliferative disorders, which include, but are not limited to diffuse large B cell lymphoma, follicular lymphoma, chronic lymphocytic lymphoma, chronic lymphocytic leukemia, B-cell prolymphocytic leukemia, lymphoplasmacytic lymphoma/Waldenström macroglobulinemia, splenic marginal zone lymphoma, plasma cell myeloma, plasmacytoma, extranodal marginal zone B cell lymphoma, nodal marginal zone B cell lymphoma, mantle cell lymphoma, mediastinal (thymic) large B cell lymphoma, intravascular large B cell lymphoma, primary effusion lymphoma, burkitt lymphoma/leukemia, and lymphomatoid granulomatosis.

In further embodiments, the methods described herein can be used to treat thromboembolic disorders, which include, but are not limited to myocardial infarct, angina pectoris (including unstable angina), reocclusions or restenoses after angioplasty or aortocoronary bypass, stroke, transitory ischemia, peripheral arterial occlusive disorders, pulmonary embolisms, and deep venous thromboses.

### Hematological Malignancies

Disclosed herein, in certain embodiments, is a method for treating a hematological malignancy in an individual in need thereof, comprising: administering to the individual a composition or tablet formulation described herein comprising an amount of Compound 1.

In some embodiments, the hematological malignancy is a non-Hodgkin's lymphoma (NHL). In some embodiments, the hematological malignancy is a chronic lymphocytic leukemia (CLL), small lymphocytic lymphoma (SLL), high risk CLL, or a non-CLL/SLL lymphoma. In some embodiments, the hematological malignancy is follicular lymphoma (FL), diffuse large B-cell lymphoma (DLBCL), mantle cell lymphoma (MCL), Waldenstrom's macroglobulinemia, multiple myeloma (MM), marginal zone lymphoma, Burkitt's lymphoma, non-Burkitt high grade B cell lymphoma, or extranodal marginal zone B cell lymphoma. In some embodiments, the hematological malignancy is acute or chronic myelogenous (or myeloid) leukemia, myelodysplastic syndrome, acute lymphoblastic leukemia, or precursor B-cell acute lymphoblastic leukemia. In some embodiments, the hematological malignancy is chronic lymphocytic leukemia (CLL). In some embodiments, the hematological malignancy is mantle cell lymphoma (MCL). In some embodiments, the hematological malignancy is diffuse large B-cell lymphoma (DLBCL). In some embodiments, the hematological malignancy is diffuse large B-cell lymphoma (DLBCL), ABC subtype. In some embodiments, the hematological malignancy is diffuse large B-cell lymphoma (DLBCL), GCB subtype. In some embodiments, the hematological malignancy is Waldenstrom's macroglobulinemia (WM). In some embodiments, the hematological malignancy is multiple myeloma (MM). In some embodiments, the hematological malignancy is Burkitt's lymphoma. In some embodiments, the hematological malignancy is follicular lymphoma (FL). In some embodiments, the hematological malignancy is transformed follicular lymphoma. In some embodiments, the hematological malignancy is marginal zone lymphoma.

In some embodiments, the hematological malignancy is relapsed or refractory non-Hodgkin's lymphoma (NHL). In some embodiments, the hematological malignancy is relapsed or refractory diffuse large B-cell lymphoma (DLBCL), relapsed or refractory mantle cell lymphoma (MCL), relapsed or refractory follicular lymphoma (FL), relapsed or refractory CLL, relapsed or refractory SLL, relapsed or refractory multiple myeloma, relapsed or refractory Waldenstrom's macroglobulinemia, relapsed or refractory multiple myeloma (MM), relapsed or refractory marginal zone lymphoma, relapsed or refractory Burkitt's lymphoma, relapsed or refractory non-Burkitt high grade B cell lymphoma, relapsed or refractory extranodal marginal zone B cell lymphoma. In some embodiments, the hematological malignancy is a relapsed or refractory acute or chronic myelogenous (or myeloid) leukemia, relapsed or refractory myelodysplastic syndrome, relapsed or refractory acute lymphoblastic leukemia, or relapsed or refractory precursor B-cell acute lymphoblastic leukemia. In some embodiments, the hematological malignancy is relapsed or refractory chronic lymphocytic leukemia (CLL). In some embodiments, the hematological malignancy is relapsed or refractory mantle cell lymphoma (MCL). In some embodiments, the hematological malignancy is relapsed or refractory diffuse large B-cell lymphoma (DLBCL). In some embodiments, the hematological malignancy is relapsed or refractory diffuse large B-cell lymphoma (DLBCL), ABC subtype. In some embodiments, the hematological malignancy is relapsed or refractory diffuse large B-cell lymphoma (DLBCL), GCB subtype. In some embodiments, the hematological malignancy is relapsed or refractory Waldenstrom's macroglobulinemia (WM). In some embodiments, the hematological malignancy is relapsed or refractory multiple myeloma (MM). In some embodiments, the hematological malignancy is relapsed or refractory Burkitt's lymphoma. In some embodiments, the hematological malignancy is relapsed or refractory follicular lymphoma (FL).

In some embodiments, the hematological malignancy is a hematological malignancy that is classified as high-risk. In some embodiments, the hematological malignancy is high risk CLL or high risk SLL.

B-cell lymphoproliferative disorders (BCLDs) are neoplasms of the blood and encompass, inter alia, non-Hodgkin lymphoma, multiple myeloma, and leukemia. BCLDs can originate either in the lymphatic tissues (as in the case of lymphoma) or in the bone marrow (as in the case of leukemia and myeloma), and they all are involved with the uncontrolled growth of lymphocytes or white blood cells. There are many subtypes of BCLD, e.g., chronic lymphocytic leukemia (CLL) and non-Hodgkin lymphoma (NHL). The disease course and treatment of BCLD is dependent on the BCLD subtype; however, even within each subtype the clinical presentation, morphologic appearance, and response to therapy is heterogeneous.

Malignant lymphomas are neoplastic transformations of cells that reside predominantly within lymphoid tissues. Two groups of malignant lymphomas are Hodgkin's lymphoma and non-Hodgkin's lymphoma (NHL). Both types of lymphomas infiltrate reticuloendothelial tissues. However, they differ in the neoplastic cell of origin, site of disease, presence of systemic symptoms, and response to treatment (Freedman et al., "Non-Hodgkin's Lymphomas" Chapter 134, Cancer Medicine, (an approved publication of the American Cancer Society, B.C. Decker Inc., Hamilton, Ontario, 2003).

### Non-Hodgkin's Lymphomas

Disclosed herein, in certain embodiments, is a method for treating a non-Hodgkin's lymphoma in an individual in need thereof, comprising: administering to the individual a composition or tablet formulation described herein comprising an amount of Compound 1.

Further disclosed herein, in certain embodiments, is a method for treating relapsed or refractory non-Hodgkin's lymphoma in an individual in need thereof, comprising: administering to the individual a therapeutically-effective amount of Compound 1. In some embodiments, the non-Hodgkin's lymphoma is relapsed or refractory diffuse large B-cell lymphoma (DLBCL), relapsed or refractory mantle cell lymphoma, relapsed or refractory follicular lymphoma, or relapsed or refractory CLL. Non-Hodgkin lymphomas (NHL) are a diverse group of malignancies that are predominately of B-cell origin. NHL may develop in any organs associated with lymphatic system such as spleen, lymph nodes or tonsils and can occur at any age. NHL is often marked by enlarged lymph nodes, fever, and weight loss. NHL is classified as either B-cell or T-cell NHL. Lymphomas related to lymphoproliferative disorders following bone marrow or stem cell transplantation are usually B-cell NHL. In the Working Formulation classification scheme, NHL has been divided into low-, intermediate-, and high-grade categories by virtue of their natural histories (see "The Non-Hodgkin's Lymphoma Pathologic Classification Project," Cancer 49(1982):2112-2135). The low-grade lymphomas are indolent, with a median survival of 5 to 10 years (Horning and Rosenberg (1984) N. Engl. J. Med. 311:1471-1475). Although chemotherapy can induce remissions in the majority of indolent lymphomas, cures are rare and most patients eventually relapse, requiring further therapy. The intermediate- and high-grade lymphomas are more aggressive tumors, but they have a greater chance for cure with chemotherapy. However, a significant proportion of these patients will relapse and require further treatment.

A non-limiting list of the B-cell NHL includes Burkitt's lymphoma (e.g., Endemic Burkitt's Lymphoma and Sporadic Burkitt's Lymphoma), Cutaneous B-Cell Lymphoma, Cutaneous Marginal Zone Lymphoma (MZL), Diffuse Large Cell Lymphoma (DLBCL), Diffuse Mixed Small and Large Cell Lymphoma, Diffuse Small Cleaved Cell, Diffuse Small Lymphocytic Lymphoma, Extranodal Marginal Zone B-cell lymphoma, follicular lymphoma, Follicular Small Cleaved Cell (Grade 1), Follicular Mixed Small Cleaved and Large Cell (Grade 2), Follicular Large Cell (Grade 3), Intravascular Large B-Cell Lymphoma, Intravascular Lymphomatosis, Large Cell Immunoblastic Lymphoma, Large Cell Lymphoma (LCL), Lymphoblastic Lymphoma, MALT Lymphoma, Mantle Cell Lymphoma (MCL), immunoblastic large cell lymphoma, precursor B-lymphoblastic lymphoma, mantle cell lymphoma, chronic lymphocytic leukemia (CLL)/small lymphocytic lymphoma (SLL), extranodal marginal zone B-cell lymphoma-mucosa-associated lymphoid tissue (MALT) lymphoma, Mediastinal Large B-Cell Lymphoma, nodal marginal zone B-cell lymphoma, splenic marginal zone B-cell lymphoma, primary mediastinal B-cell lymphoma, lymphoplasmocytic lymphoma, hairy cell leukemia, Waldenstrom's Macroglobulinemia, and primary central nervous system (CNS) lymphoma. Additional non-Hodgkin's lymphomas are contemplated within the scope of the present invention and apparent to those of ordinary skill in the art.

### DLBCL

Disclosed herein, in certain embodiments, is a method for treating a DLCBL in an individual in need thereof, comprising: administering to the individual a composition or tablet formulation described herein comprising an amount of Compound 1. Further disclosed herein, in certain embodiments, is a method for treating relapsed or refractory DLCBL in an individual in need thereof, comprising: administering to the individual a composition or tablet formulation described herein comprising a therapeutically-effective amount of Compound 1.

As used herein, the term "Diffuse large B-cell lymphoma (DLBCL)" refers to a neoplasm of the germinal center B lymphocytes with a diffuse growth pattern and a high-intermediate proliferation index. DLBCLs represent approximately 30% of all lymphomas and may present with several morphological variants including the centroblastic, immunoblastic, T-cell/histiocyte rich, anaplastic and plasmoblastic subtypes. Genetic tests have shown that there are different subtypes of DLBCL. These subtypes seem to have different outlooks (prognoses) and responses to treatment. DLBCL can affect any age group but occurs mostly in older people (the average age is mid-60s).

Disclosed herein, in certain embodiments, is a method for treating diffuse large B-cell lymphoma, activated B cell-like subtype (ABC-DLBCL), in an individual in need thereof, comprising: administering to the individual an irreversible Btk inhibitor in an amount from 300 mg/day up to, and including, 1000 mg/day. The ABC subtype of diffuse large B-cell lymphoma (ABC-DLBCL) is thought to arise from post germinal center B cells that are arrested during plasmatic differentiation. The ABC subtype of DLBCL (ABC-DLBCL) accounts for approximately 30% total DLBCL diagnoses. It is considered the least curable of the DLBCL molecular subtypes and, as such, patients diagnosed with the ABC-DLBCL typically display significantly reduced survival rates compared with individuals with other types of DLCBL. ABC-DLBCL is most commonly associated with chromosomal translocations deregulating the germinal center master regulator BCL6 and with mutations inactivating the PRDM1 gene, which encodes a transcriptional repressor required for plasma cell differentiation.

A particularly relevant signaling pathway in the pathogenesis of ABC-DLBCL is the one mediated by the nuclear factor (NF)-κB transcription complex. The NF-κB family comprises 5 members (p50, p52, p65, c-rel and RelB) that form homo- and heterodimers and function as transcriptional factors to mediate a variety of proliferation, apoptosis, inflammatory and immune responses and are critical for normal B-cell development and survival. NF-κB is widely used by eukaryotic cells as a regulator of genes that control cell proliferation and cell survival. As such, many different types of human tumors have misregulated NF-κB: that is, NF-κB is constitutively active. Active NF-κB turns on the expression of genes that keep the cell proliferating and protect the cell from conditions that would otherwise cause it to die via apoptosis.

The dependence of ABC DLBCLs on NF-kB depends on a signaling pathway upstream of IkB kinase comprised of CARD11, BCL10 and MALT1 (the CBM complex). Interference with the CBM pathway extinguishes NF-kB signaling in ABC DLBCL cells and induces apoptosis. The molecular basis for constitutive activity of the NF-kB pathway is a subject of current investigation but some somatic alterations to the genome of ABC DLBCLs clearly invoke this pathway. For example, somatic mutations of the coiled-coil domain of CARD11 in DLBCL render this signaling scaffold protein able to spontaneously nucleate protein-protein interaction with MALT1 and BCL10, causing IKK activity and NF-kB activation. Constitutive activity of the B cell receptor signaling pathway has been implicated in the activation of NF-kB in ABC DLBCLs with wild type CARD11, and this is associated with mutations within the cytoplasmic tails of the B cell receptor subunits CD79A and CD79B. Oncogenic activating mutations in the signaling adapter MYD88 activate NF-kB and synergize with B cell receptor signaling in sustaining the survival of ABC DLBCL cells. In addition, inactivating mutations in a negative regulator of the NF-kB pathway, A20, occur almost exclusively in ABC DLBCL.

Indeed, genetic alterations affecting multiple components of the NF-κB signaling pathway have been recently identified in more than 50% of ABC-DLBCL patients, where these lesions promote constitutive NF-κB activation, thereby contributing to lymphoma growth. These include mutations of CARD11 (~10% of the cases), a lymphocyte-specific cytoplasmic scaffolding protein that-together with MALT1 and BCL10-forms the BCR signalosome, which relays signals from antigen receptors to the downstream mediators of NF-κB activation. An even larger fraction of cases (~30%) carry biallelic genetic lesions inactivating the negative NF-κB regulator A20. Further, high levels of expression of NF-κB target genes have been observed in ABC-DLBCL tumor samples. *See, e.g.,* U. Klein et al., (2008), Nature Reviews Immunology 8:22-23; R.E. Davis et al., (2001), Journal of Experimental Medicine 194:1861-1874; G. Lentz et al., (2008), Science 319:1676-1679; M. Compagno et al., (2009), Nature 459:712-721; and L. Srinivasan et al., (2009), Cell 139:573-586).

DLBCL cells of the ABC subtype, such as OCI-Ly10, have chronic active BCR signaling and are very sensitive to the Btk inhibitor described herein. The irreversible Btk inhibitor described herein potently and irreversibly inhibits the growth of OCI-Ly10 (EC₅₀ continuous exposure = 10 nM, EC₅₀ 1 hour pulse = 50 nM). In addition, induction of apoptosis, as shown by capsase activation, Annexin-V flow cytometry and increase in sub-GO fraction is observed in OCILy10. Both sensitive and resistant cells express Btk at similar levels, and the active site of Btk is fully occupied by the inhibitor in both as shown using a fluorescently labeled affinity probe. OCI-Ly10 cells are shown to have chronically active BCR signaling to NF-kB which is dose dependently inhibited by the Btk inhibitors described herein. The activity of Btk inhibitors in the cell lines studied herein are also characterized by comparing signal transduction profiles (Btk, PLCγ, ERK, NF-kB, AKT), cytokine secretion profiles and mRNA expression profiles, both with and without BCR stimulation, and observed significant differences in these profiles that lead to clinical biomarkers that identify the most sensitive patient populations to Btk inhibitor treatment. *See* U.S. Patent No. 7,711,492 and Staudt et al., Nature, Vol. 463, Jan. 7, 2010, pp. 88-92.

### Follicular Lymphoma

Disclosed herein, in certain embodiments, is a method for treating a follicular lymphoma in an individual in need thereof, comprising: administering to the individual a composition or tablet formulation described herein comprising an amount of Compound 1. Further disclosed herein, in certain embodiments, is a method for treating relapsed or refractory follicular lymphoma in an individual in need thereof, comprising: administering to the individual a composition or tablet formulation described herein comprising a therapeutically-effective amount of Compound 1.

As used herein, the term "follicular lymphoma" refers to any of several types of non-Hodgkin's lymphoma in which the lymphomatous cells are clustered into nodules or follicles. The term follicular is used because the cells tend to grow in a circular, or nodular, pattern in lymph nodes. The average age for people with this lymphoma is about 60.

### CLL/SLL

Disclosed herein, in certain embodiments, is a method for treating a CLL or SLL in an individual in need thereof, comprising: administering to the individual a composition or tablet formulation described herein comprising an amount of Compound 1. Further disclosed herein, in certain embodiments, is a method for treating relapsed or refractory CLL or SLL in an individual in need thereof, comprising: administering to the individual a composition or tablet formulation described herein comprising a therapeutically-effective amount of Compound 1.

Chronic lymphocytic leukemia and small lymphocytic lymphoma (CLL/SLL) are commonly thought as the same disease with slightly different manifestations. Where the cancerous cells gather determines whether it is called CLL or SLL. When the cancer cells are primarily found in the lymph nodes, lima bean shaped structures of the lymphatic system (a system primarily of tiny vessels found in the body), it is called SLL. SLL accounts for about 5% to 10% of all lymphomas. When most of the cancer cells are in the bloodstream and the bone marrow, it is called CLL.

Both CLL and SLL are slow-growing diseases, although CLL, which is much more common, tends to grow slower. CLL and SLL are treated the same way. They are usually not considered curable with standard treatments, but depending on the stage and growth rate of the disease, most patients live longer than 10 years. Occasionally over time, these slow-growing lymphomas may transform into a more aggressive type of lymphoma.

Chronic lymphoid leukemia (CLL) is the most common type of leukemia. It is estimated that 100,760 people in the United States are living with or are in remission from CLL. Most (>75%) people newly diagnosed with CLL are over the age of 50. Currently CLL treatment focuses on controlling the disease and its symptoms rather than on an outright cure. CLL is treated by chemotherapy, radiation therapy, biological therapy, or bone marrow transplantation. Symptoms are sometimes treated surgically (splenectomy removal of enlarged spleen) or by radiation therapy ("de-bulking" swollen lymph nodes). Though CLL progresses slowly in most cases, it is considered generally incurable. Certain CLLs are classified as high-risk. As used herein, "high risk CLL" means CLL characterized by at least one of the following 1) 17p13-; 2) 11q22-; 3) unmutated IgVH together with ZAP-70+ and/or CD38+; or 4) trisomy 12.

CLL treatment is typically administered when the patient's clinical symptoms or blood counts indicate that the disease has progressed to a point where it may affect the patient's quality of life.

Small lymphocytic leukemia (SLL) is very similar to CLL described supra, and is also a cancer of B-cells. In SLL the abnormal lymphocytes mainly affect the lymph nodes. However, in CLL the abnormal cells mainly affect the blood and the bone marrow. The spleen may be affected in both conditions. SLL accounts for about 1 in 25 of all cases of non-Hodgkin lymphoma. It can occur at any time from young adulthood to old age, but is rare under the age of 50. SLL is considered an indolent lymphoma. This means that the disease progresses very slowly, and patients tend to live many years after diagnosis. However, most patients are diagnosed with advanced disease, and although SLL responds well to a variety of chemotherapy drugs, it is generally considered to be incurable. Although some cancers tend to occur more often in one gender or the other, cases and deaths due to SLL are evenly split between men and women. The average age at the time of diagnosis is 60 years.

Although SLL is indolent, it is persistently progressive. The usual pattern of this disease is one of high response rates to radiation therapy and/or chemotherapy, with a period of disease remission. This is followed months or years later by an inevitable relapse. Re-treatment leads to a response again, but again the disease will relapse. This means that although the short-term prognosis of SLL is quite good, over time, many patients develop fatal complications of recurrent disease. Considering the age of the individuals typically diagnosed with CLL and SLL, there is a need in the art for a simple and effective treatment of the disease with minimum side-effects that do not impede on the patient's quality of life. The instant invention fulfills this long standing need in the art.

### Mantle Cell Lymphoma

Disclosed herein, in certain embodiments, is a method for treating a Mantle cell lymphoma in an individual in need thereof, comprising: administering to the individual a composition or tablet formulation described herein comprising an amount of Compound 1. Further disclosed herein, in certain embodiments, is a method for treating relapsed or refractory Mantle cell lymphoma in an individual in need thereof, comprising: administering to the individual a composition or tablet formulation described herein comprising a therapeutically-effective amount of Compound 1.

As used herein, the term, "Mantle cell lymphoma" refers to a subtype of B-cell lymphoma, due to CD5 positive antigen-naive pregerminal center B-cell within the mantle zone that surrounds normal germinal center follicles. MCL cells generally over-express cyclin D1 due to a t(11:14) chromosomal translocation in the DNA. More specifically, the translocation is at t(11;14)(q13;q32). Only about 5% of lymphomas are of this type. The cells are small to medium in size. Men are affected most often. The average age of patients is in the early 60s. The lymphoma is usually widespread when it is diagnosed, involving lymph nodes, bone marrow, and, very often, the spleen. Mantle cell lymphoma is not a very fast growing lymphoma, but is difficult to treat.

### Marginal Zone B-cell Lymphoma

Disclosed herein, in certain embodiments, is a method for treating a marginal zone B-cell lymphoma in an individual in need thereof, comprising: administering to the individual a composition or tablet formulation described herein comprising an amount of Compound 1. Further disclosed herein, in certain embodiments, is a method for treating relapsed or refractory marginal zone B-cell lymphoma in an individual in need thereof, comprising: administering to the individual a composition or tablet formulation described herein comprising a therapeutically-effective amount of Compound 1.

As used herein, the term "marginal zone B-cell lymphoma" refers to a group of related B-cell neoplasms that involve the lymphoid tissues in the marginal zone, the patchy area outside the follicular mantle zone. Marginal zone lymphomas account for about 5% to 10% of lymphomas. The cells in these lymphomas look small under the microscope. There are 3 main types of marginal zone lymphomas including extranodal marginal zone B-cell lymphomas, nodal marginal zone B-cell lymphoma, and splenic marginal zone lymphoma.

### MALT

Disclosed herein, in certain embodiments, is a method for treating a MALT in an individual in need thereof, comprising: administering to the individual an amount of Compound 1. Further disclosed herein, in certain embodiments, is a method for treating relapsed or refractory MALT in an individual in need thereof, comprising: administering to the individual a composition or tablet formulation described herein comprising a therapeutically-effective amount of Compound 1.

The term "mucosa-associated lymphoid tissue (MALT) lymphoma", as used herein, refers to extranodal manifestations of marginal-zone lymphomas. Most MALT lymphoma are a low grade, although a minority either manifest initially as intermediate-grade non-Hodgkin lymphoma (NHL) or evolve from the low-grade form. Most of the MALT lymphoma occur in the stomach, and roughly 70% of gastric MALT lymphoma are associated with Helicobacter pylori infection. Several cytogenetic abnormalities have been identified, the most common being trisomy 3 or t(11;18). Many of these other MALT lymphoma have also been linked to infections with bacteria or viruses. The average age of patients with MALT lymphoma is about 60.

### Nodal Marginal Zone B-Cell Lymphoma

Disclosed herein, in certain embodiments, is a method for treating a nodal marginal zone B-cell lymphoma in an individual in need thereof, comprising: administering to the individual a composition or tablet formulation described herein comprising an amount of Compound 1. Further disclosed herein, in certain embodiments, is a method for treating relapsed or refractory nodal marginal zone B-cell lymphoma in an individual in need thereof, comprising: administering to the individual a composition or tablet formulation described herein comprising a therapeutically-effective amount of Compound 1.

The term "nodal marginal zone B-cell lymphoma" refers to an indolent B-cell lymphoma that is found mostly in the lymph nodes. The disease is rare and only accounts for 1% of all Non-Hodgkin's Lymphomas (NHL). It is most commonly diagnosed in older patients, with women more susceptible than men. The disease is classified as a marginal zone lymphoma because the mutation occurs in the marginal zone of the B-cells. Due to its confinement in the lymph nodes, this disease is also classified as nodal.

### Splenic Marginal Zone B-Cell Lymphoma

Disclosed herein, in certain embodiments, is a method for treating a splenic marginal zone B-cell lymphoma in an individual in need thereof, comprising: administering to the individual a composition or tablet formulation described herein comprising an amount of Compound 1. Further disclosed herein, in certain embodiments, is a method for treating relapsed or refractory splenic marginal zone B-cell lymphoma in an individual in need thereof, comprising: administering to the individual a composition or tablet formulation described herein comprising a therapeutically-effective amount of Compound 1.

The term "splenic marginal zone B-cell lymphoma" refers to specific low-grade small B-cell lymphoma that is incorporated in the World Health Organization classification. Characteristic features are splenomegaly, moderate lymphocytosis with villous morphology, intrasinusoidal pattern of involvement of various organs, especially bone marrow, and relative indolent course. Tumor progression with increase of blastic forms and aggressive behavior are observed in a minority of patients. Molecular and cytogenetic studies have shown heterogeneous results probably because of the lack of standardized diagnostic criteria.

### Burkitt Lymphoma

Disclosed herein, in certain embodiments, is a method for treating a Burkitt lymphoma in an individual in need thereof, comprising: administering to the individual a composition or tablet formulation described herein comprising an amount of Compound 1. Further disclosed herein, in certain embodiments, is a method for treating relapsed or refractory Burkitt lymphoma in an individual in need thereof, comprising: administering to the individual a composition or tablet formulation described herein comprising a therapeutically-effective amount of Compound 1.

The term "Burkitt lymphoma" refers to a type of Non-Hodgkin Lymphoma (NHL) that commonly affects children. It is a highly aggressive type of B-cell lymphoma that often starts and involves body parts other than lymph nodes. In spite of its fast-growing nature, Burkitt's lymphoma is often curable with modern intensive therapies. There are two broad types of Burkitt's lymphoma - the sporadic and the endemic varieties: Endemic Burkitt's lymphoma: The disease involves children much more than adults, and is related to Epstein Barr Virus (EBV) infection in 95% cases. It occurs primarily is equatorial Africa, where about half of all childhood cancers are Burkitt's lymphoma. It characteristically has a high chance of involving the jawbone, a rather distinctive feature that is rare in sporadic Burkitt's. It also commonly involves the abdomen. Sporadic Burkitt's lymphoma: The type of Burkitt's lymphoma that affects the rest of the world, including Europe and the Americas is the sporadic type. Here too, it's mainly a disease in children. The link between Epstein Barr Virus (EBV) is not as strong as with the endemic variety, though direct evidence of EBV infection is present in one out of five patients. More than the involvement of lymph nodes, it is the abdomen that is notably affected in more than 90% of the children. Bone marrow involvement is more common than in the sporadic variety.

### Waldenstrom Macroglobulinemia

Disclosed herein, in certain embodiments, is a method for treating a Waldenstrom macroglobulinemia in an individual in need thereof, comprising: administering to the individual a composition or tablet formulation described herein comprising an amount of Compound 1. Further disclosed herein, in certain embodiments, is a method for treating relapsed or refractory Waldenstrom macroglobulinemia in an individual in need thereof, comprising: administering to the individual a composition or tablet formulation described herein comprising a therapeutically-effective amount of Compound 1.

The term "Waldenstrom macroglobulinemia", also known as lymphoplasmacytic lymphoma, is cancer involving a subtype of white blood cells called lymphocytes. It is characterized by an uncontrolled clonal proliferation of terminally differentiated B lymphocytes. It is also characterized by the lymphoma cells making an antibody called immunoglobulin M (IgM). The IgM antibodies circulate in the blood in large amounts, and cause the liquid part of the blood to thicken, like syrup. This can lead to decreased blood flow to many organs, which can cause problems with vision (because of poor circulation in blood vessels in the back of the eyes) and neurological problems (such as headache, dizziness, and confusion) caused by poor blood flow within the brain. Other symptoms can include feeling tired and weak, and a tendency to bleed easily. The underlying etiology is not fully understood but a number of risk factors have been identified, including the locus 6p21.3 on chromosome 6. There is a 2- to 3-fold risk increase of developing WM in people with a personal history of autoimmune diseases with autoantibodies and particularly elevated risks associated with hepatitis, human immunodeficiency virus, and rickettsiosis.

### Multiple Myeloma

Disclosed herein, in certain embodiments, is a method for treating a myeloma in an individual in need thereof, comprising: administering to the individual a composition or tablet formulation described herein comprising an amount of Compound 1. Further disclosed herein, in certain embodiments, is a method for treating relapsed or refractory myeloma in an individual in need thereof, comprising: administering to the individual a composition or tablet formulation described herein comprising a therapeutically-effective amount of Compound 1.

Multiple myeloma, also known as MM, myeloma, plasma cell myeloma, or as Kahler's disease (after Otto Kahler) is a cancer of the white blood cells known as plasma cells. A type of B cell, plasma cells are a crucial part of the immune system responsible for the production of antibodies in humans and other vertebrates. They are produced in the bone marrow and are transported through the lymphatic system.

### Leukemia

Disclosed herein, in certain embodiments, is a method for treating a leukemia in an individual in need thereof, comprising: administering to the individual a composition or tablet formulation described herein comprising an amount of Compound 1. Further disclosed herein, in certain embodiments, is a method for treating relapsed or refractory leukemia in an individual in need thereof, comprising: administering to the individual a composition or tablet formulation described herein comprising a therapeutically-effective amount of Compound 1.

Leukemia is a cancer of the blood or bone marrow characterized by an abnormal increase of blood cells, usually leukocytes (white blood cells). Leukemia is a broad term covering a spectrum of diseases. The first division is between its acute and chronic forms: (i) acute leukemia is characterized by the rapid increase of immature blood cells. This crowding makes the bone marrow unable to produce healthy blood cells. Immediate treatment is required in acute leukemia due to the rapid progression and accumulation of the malignant cells, which then spill over into the bloodstream and spread to other organs of the body. Acute forms of leukemia are the most common forms of leukemia in children; (ii) chronic leukemia is distinguished by the excessive build up of relatively mature, but still abnormal, white blood cells. Typically taking months or years to progress, the cells are produced at a much higher rate than normal cells, resulting in many abnormal white blood cells in the blood. Chronic leukemia mostly occurs in older people, but can theoretically occur in any age group. Additionally, the diseases are subdivided according to which kind of blood cell is affected. This split divides leukemias into lymphoblastic or lymphocytic leukemias and myeloid or myelogenous leukemias: (i) lymphoblastic or lymphocytic leukemias, the cancerous change takes place in a type of marrow cell that normally goes on to form lymphocytes, which are infection-fighting immune system cells; (ii) myeloid or myelogenous leukemias, the cancerous change takes place in a type of marrow cell that normally goes on to form red blood cells, some other types of white cells, and platelets. Within these main categories, there are several subcategories including, but not limited to, Acute lymphoblastic leukemia (ALL), precursor B-cell acute lymphoblastic leukemia (precursor B-ALL; also called precursor B-lymphoblastic leukemia), Acute myelogenous leukemia (AML), Chronic myelogenous leukemia (CML), and Hairy cell leukemia (HCL). Accordingly, disclosed herein, in certain embodiments, is a method for treating Acute lymphoblastic leukemia (ALL), precursor B-cell acute lymphoblastic leukemia (precursor B-ALL; also called precursor B-lymphoblastic leukemia), Acute myelogenous leukemia (AML), Chronic myelogenous leukemia (CML), or Hairy cell leukemia (HCL) in an individual in need thereof, comprising: administering to the individual an amount of Compound 1. In some embodiments, the leukemia is a relapsed or refractory leukemia. In some embodiments, the leukemia is a relapsed or refractory Acute lymphoblastic leukemia (ALL), relapsed or refractory precursor B-cell acute lymphoblastic leukemia (precursor B-ALL; also called precursor B-lymphoblastic leukemia), relapsed or refractory Acute myelogenous leukemia (AML), relapsed or refractory Chronic myelogenous leukemia (CML), or relapsed or refractory Hairy cell leukemia (HCL).

Symptoms, diagnostic tests, and prognostic tests for each of the above-mentioned conditions are known. See, e.g., Harrison's Principles of InternalMedicine©," 16th ed., 2004, The McGraw-Hill Companies, Inc. Dey et al. (2006), Cytojournal 3(24), and the "Revised European American Lymphoma" (REAL) classification system (see, e.g., the website maintained by the National Cancer Institute).

A number of animal models of are useful for establishing a range of therapeutically effective doses of irreversible Btk inhibitor compounds, such as Compound 1, for treating any of the foregoing diseases.

The therapeutic efficacy of Compound 1 for any one of the foregoing diseases can be optimized during a course of treatment. For example, a subject being treated can undergo a diagnostic evaluation to correlate the relief of disease symptoms or pathologies to inhibition of *in vivo* Btk activity achieved by administering a given dose of Compound 1. Cellular assays known in the art can be used to determine *in vivo* activity of Btk in the presence or absence of an irreversible Btk inhibitor. For example, since activated Btk is phosphorylated at tyrosine 223 (Y223) and tyrosine 551 (Y551), phospho-specific immunocytochemical staining of P-Y223 or P-Y551-positive cells can be used to detect or quantify activation of Btk in a population of cells (e.g., by FACS analysis of stained vs unstained cells). See, e.g., Nisitani et al. (1999), Proc. Natl. Acad. Sci, USA 96:2221-2226. Thus, the amount of the Btk inhibitor compound that is administered to a subject can be increased or decreased as needed so as to maintain a level of Btk inhibition optimal for treating the subject's disease state. Compound 1 can irreversibly inhibit Btk and may be used to treat mammals suffering from Bruton's tyrosine kinase-dependent or Bruton's tyrosine kinase mediated conditions or diseases, including, but not limited to, cancer, autoimmune and other inflammatory diseases. Compound 1 has shown efficacy is a wide variety of diseases and conditions that are described herein.

In some embodiments, Compound 1 is used for the manufacture of a medicament for treating any of the foregoing conditions (e.g., autoimmune diseases, inflammatory diseases, allergy disorders, B-cell proliferative disorders, or thromboembolic disorders).

### Compound 1, and Pharmaceutically Acceptable Salts Thereof

The Btk inhibitor compound described herein (i.e. Compound 1) is selective for Btk and kinases having a cysteine residue in an amino acid sequence position of the tyrosine kinase that is homologous to the amino acid sequence position of cysteine 481 in Btk. The Btk inhibitor compound can form a covalent bond with Cys 481 of Btk (e.g., via a Michael reaction).

"Compound 1" or "1-((R)-3-(4-amino-3-(4-phenoxyphenyl)-1H-pyrazolo[3,4-d]pyrimidin-1-yl)piperidin-1-yl)prop-2-en-1-one" or "1-{(3*R*)-3-[4-amino-3-(4-phenoxyphenyl)-1*H*-pyrazolo[3,4-*d*]pyrimidin-1-yl]piperidin-1-yl-yl}prop-2-en-1-one" or "2-Propen-1-one, 1-[(3*R*)-3-[4-amino-3-(4-phenoxyphenyl)-1*H*-pyrazolo[3,4-*d*]pyrimidin-1-yl]-1-piperidinyl-" or ibrutinib or any other suitable name refers to the compound with the following structure:

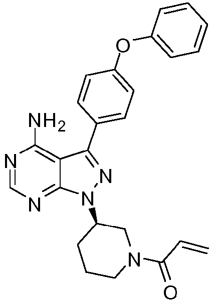

A wide variety of pharmaceutically acceptable salts is formed from Compound 1 and includes:
- acid addition salts formed by reacting Compound 1 with an organic acid, which includes aliphatic mono- and dicarboxylic acids, phenyl-substituted alkanoic acids, hydroxyl alkanoic acids, alkanedioic acids, aromatic acids, aliphatic and aromatic sulfonic acids, amino acids, etc. and include, for example, acetic acid, trifluoroacetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like;
- acid addition salts formed by reacting Compound 1 with an inorganic acid, which includes hydrochloric acid, hydrobromic acid, sulfuric acid, nitric acid, phosphoric acid, hydroiodic acid, hydrofluoric acid, phosphorous acid, and the like.

The term "pharmaceutically acceptable salts" in reference to Compound 1 refers to a salt of Compound 1, which does not cause significant irritation to a mammal to which it is administered and does not substantially abrogate the biological activity and properties of the compound.

It should be understood that a reference to a pharmaceutically acceptable salt includes the solvent addition forms (solvates). Solvates contain either stoichiometric or non-stoichiometric amounts of a solvent, and are formed during the process of product formation or isolation with pharmaceutically acceptable solvents such as water, ethanol, methanol, methyl tert-butyl ether (MTBE), diisopropyl ether (DIPE), ethyl acetate, isopropyl acetate, isopropyl alcohol, methyl isobutyl ketone (MIBK), methyl ethyl ketone (MEK), acetone, nitromethane, tetrahydrofuran (THF), dichloromethane (DCM), dioxane, heptanes, toluene, anisole, acetonitrile, and the like. In one aspect, solvates are formed using, but not limited to, Class 3 solvent(s). Categories of solvents are defined in, for example, the International Conference on Harmonization of Technical Requirements for Registration of Pharmaceuticals for Human Use (ICH), "Impurities: Guidelines for Residual Solvents, Q3C(R3), (November 2005). Hydrates are formed when the solvent is water, or alcoholates are formed when the solvent is alcohol. In some embodiments, solvates of Compound 1, or pharmaceutically acceptable salts thereof, are conveniently prepared or formed during the processes described herein. In some embodiments, solvates of Compound 1 are anhydrous. In some embodiments, Compound 1, or pharmaceutically acceptable salts thereof, exist in unsolvated form. In some embodiments, Compound 1, or pharmaceutically acceptable salts thereof, exist in unsolvated form and are anhydrous.

In yet other embodiments, Compound 1, or a pharmaceutically acceptable salt thereof, is prepared in various forms, including but not limited to, amorphous phase, crystalline forms, milled forms and nano-particulate forms. In some embodiments, Compound 1, or a pharmaceutically acceptable salt thereof, is amorphous. In some embodiments, Compound 1, or a pharmaceutically acceptable salt thereof, is amorphous and anhydrous. In some embodiments, Compound 1, or a pharmaceutically acceptable salt thereof, is crystalline. In some embodiments, Compound 1, or a pharmaceutically acceptable salt thereof, is crystalline and anhydrous.

In some embodiments, Compound 1 is prepared as outlined in US Patent no. 7,514,444.

### Certain Terminology

Unless defined otherwise, all technical and scientific terms used herein have the same meaning as is commonly understood by one of skill in the art to which the claimed subject matter belongs. It is to be understood that the foregoing general description and the following detailed description are exemplary and explanatory only and are not restrictive of any subject matter claimed. In this application, the use of the singular includes the plural unless specifically stated otherwise. It must be noted that, as used in the specification and the appended claims, the singular forms "a," "an" and "the" include plural referents unless the context clearly dictates otherwise. In this application, the use of "or" means "and/or" unless stated otherwise. Furthermore, use of the term "including" as well as other forms, such as "include", "includes," and "included," is not limiting.

The section headings used herein are for organizational purposes only and are not to be construed as limiting the subject matter described.

The term "about" when used before a numerical value indicates that the value may vary within a reasonable range, such as within ± 10%, ±5% or ±1% of the stated value.

As used herein, the term "comprising" is intended to mean that the compositions and methods, etc., include the recited elements, but do not exclude others. "Consisting essentially of" when used to define compositions and methods, shall mean excluding other elements of any essential significance to the combination for the intended use, but not excluding elements that do not materially affect the characteristic(s) of the compositions or methods. "Consisting of" shall mean excluding elements not specifically recited. Embodiments defined by each of these transition terms are within the scope of this invention.

The term "acceptable" or "pharmaceutically acceptable", with respect to a formulation, composition or ingredient, as used herein, means having no persistent detrimental effect on the general health of the subject being treated or does not abrogate the biological activity or properties of the compound, and is relatively nontoxic.

As used herein, the term "agonist" refers to a compound, the presence of which results in a biological activity of a protein that is the same as the biological activity resulting from the presence of a naturally occurring ligand for the protein, such as, for example, Btk.

As used herein, the term "partial agonist" refers to a compound the presence of which results in a biological activity of a protein that is of the same type as that resulting from the presence of a naturally occurring ligand for the protein, but of a lower magnitude. As used herein, the term "antagonist" refers to a compound, the presence of which results in a decrease in the magnitude of a biological activity of a protein. In certain embodiments, the presence of an antagonist results in complete inhibition of a biological activity of a protein, such as, for example, Btk. In certain embodiments, an antagonist is an inhibitor.

As used herein, "amelioration" of the symptoms of a particular disease, disorder or condition by administration of a particular compound or pharmaceutical composition refers to any lessening of severity, delay in onset, slowing of progression, or shortening of duration, whether permanent or temporary, lasting or transient that can be attributed to or associated with administration of the compound or composition.

"Bioavailability" refers to the percentage of Compound 1 dosed that is delivered into the general circulation of the animal or human being studied. The total exposure (AUC_{(0-∞)}) of a drug when administered intravenously is usually defined as 100% bioavailable (F%). "Oral bioavailability" refers to the extent to which Compound 1 is absorbed into the general circulation when the pharmaceutical composition is taken orally as compared to intravenous injection.

"Blood plasma concentration" refers to the concentration of Compound 1 in the plasma component of blood of a subject. It is understood that the plasma concentration of Compound 1 may vary significantly between subjects, due to variability with respect to metabolism and/or possible interactions with other therapeutic agents. In accordance with one embodiment disclosed herein, the blood plasma concentration of Compound 1 may vary from subject to subject. Likewise, values such as maximum plasma concentration (Cₘₐₓ) or time to reach maximum plasma concentration (Tₘₐₓ), or total area under the plasma concentration time curve (AUC_{(0-∞)}) may vary from subject to subject. Due to this variability, the amount necessary to constitute "a therapeutically effective amount" of Compound 1 may vary from subject to subject.

The term "Bruton's tyrosine kinase," as used herein, refers to Bruton's tyrosine kinase from *Homo sapiens,* as disclosed in, e.g., U.S. Patent No. 6,326,469 (GenBank Accession No. NP_000052).

The terms "co-administration" or the like, as used herein, are meant to encompass administration of the selected therapeutic agents to a single patient, and are intended to include treatment regimens in which the agents are administered by the same or different route of administration or at the same or different time.

The terms "effective amount" or "therapeutically effective amount," as used herein, refer to a sufficient amount of an agent or a compound being administered which will relieve to some extent one or more of the symptoms of the disease or condition being treated. The result can be reduction and/or alleviation of the signs, symptoms, or causes of a disease, or any other desired alteration of a biological system. For example, an "effective amount" for therapeutic uses is the amount of the composition including a compound as disclosed herein required to provide a clinically significant decrease in disease symptoms without undue adverse side effects. An appropriate "effective amount" in any individual case may be determined using techniques, such as a dose escalation study. The term "therapeutically effective amount" includes, for example, a prophylactically effective amount. An "effective amount" of a compound disclosed herein is an amount effective to achieve a desired pharmacologic effect or therapeutic improvement without undue adverse side effects. It is understood that "an effect amount" or "a therapeutically effective amount" can vary from subject to subject, due to variation in metabolism of Compound 1, age, weight, general condition of the subject, the condition being treated, the severity of the condition being treated, and the judgment of the prescribing physician. By way of example only, therapeutically effective amounts may be determined by routine experimentation, including but not limited to a dose escalation clinical trial.

The terms "enhance" or "enhancing" means to increase or prolong either in potency or duration a desired effect. By way of example, "enhancing" the effect of therapeutic agents refers to the ability to increase or prolong, either in potency or duration, the effect of therapeutic agents on during treatment of a disease, disorder or condition. An "enhancing-effective amount," as used herein, refers to an amount adequate to enhance the effect of a therapeutic agent in the treatment of a disease, disorder or condition. When used in a patient, amounts effective for this use will depend on the severity and course of the disease, disorder or condition, previous therapy, the patient's health status and response to the drugs, and the judgment of the treating physician.

The terms "inhibits", "inhibiting", or "inhibitor" of a kinase, as used herein, refer to inhibition of enzymatic phosphotransferase activity.

The term "irreversible inhibitor," as used herein, refers to a compound that, upon contact with a target protein (e.g., a kinase) causes the formation of a new covalent bond with or within the protein, whereby one or more of the target protein's biological activities (e.g., phosphotransferase activity) is diminished or abolished notwithstanding the subsequent presence or absence of the irreversible inhibitor.

The term "irreversible Btk inhibitor," as used herein, refers to an inhibitor of Btk that can form a covalent bond with an amino acid residue of Btk. In one embodiment, the irreversible inhibitor of Btk can form a covalent bond with a Cys residue of Btk; in particular embodiments, the irreversible inhibitor can form a covalent bond with a Cys 481 residue (or a homolog thereof) of Btk or a cysteine residue in the homologous corresponding position of another tyrosine kinase.

The term "modulate," as used herein, means to interact with a target either directly or indirectly so as to alter the activity of the target, including, by way of example only, to enhance the activity of the target, to inhibit the activity of the target, to limit the activity of the target, or to extend the activity of the target.

As used herein, the term "modulator" refers to a compound that alters an activity of a molecule. For example, a modulator can cause an increase or decrease in the magnitude of a certain activity of a molecule compared to the magnitude of the activity in the absence of the modulator. In certain embodiments, a modulator is an inhibitor, which decreases the magnitude of one or more activities of a molecule. In certain embodiments, an inhibitor completely prevents one or more activities of a molecule. In certain embodiments, a modulator is an activator, which increases the magnitude of at least one activity of a molecule. In certain embodiments the presence of a modulator results in an activity that does not occur in the absence of the modulator.

The term "prophylactically effective amount," as used herein, refers that amount of a composition applied to a patient which will relieve to some extent one or more of the symptoms of a disease, condition or disorder being treated. In such prophylactic applications, such amounts may depend on the patient's state of health, weight, and the like. It is considered well within the skill of the art for one to determine such prophylactically effective amounts by routine experimentation, including, but not limited to, a dose escalation clinical trial.

The term "individual," "subject" or "patient" as used herein, refers to an animal which is the object of treatment, observation or experiment. By way of example only, a subject may be, but is not limited to, a mammal including, but not limited to, a human.

The term "wet granulation" as used herein, refers to the formation of granules using a granulation liquid (water, organic solvent, or a solution).

The term "dry granulation" as used herein, refers to the formation of granules without using a granulation liquid (water, organic solvent, or a solution).

The term "high-load solid tablet formulation" as used herein, refers to a solid tablet formulation comprising at least 50% w/w of ibrutinib per tablet.

As used herein, the IC₅₀ refers to an amount, concentration or dosage of a particular test compound that achieves a 50% inhibition of a maximal response, such as inhibition of Btk, in an assay that measures such response.

As used herein, EC₅₀ refers to a dosage, concentration or amount of a particular test compound that elicits a dose-dependent response at 50% of maximal expression of a particular response that is induced, provoked or potentiated by the particular test compound.

### Pharmaceutical Compositions/Formulations

A pharmaceutical composition or pharmaceutical formulation, as used herein, refers to a mixture of Compound 1 with other chemical components as described herein, such as carriers, stabilizers, diluents, dispersing agents, suspending agents, thickening agents, and/or excipients (as applicable). The pharmaceutical composition facilitates administration of the compound to a mammal. The compounds can be used singly or in combination with one or more therapeutic agents as components of mixtures.

The term "pharmaceutical combination" as used herein, means a product that results from the mixing or combining of more than one active ingredient and includes both fixed and non-fixed combinations of the active ingredients. The term "fixed combination" means that the active ingredients, e.g. Compound 1 and a co-agent, are both administered to a patient simultaneously in the form of a single entity or dosage. The term "non-fixed combination" means that the active ingredients, e.g. Compound 1 and a co-agent, are administered to a patient as separate entities either simultaneously, concurrently or sequentially with no specific intervening time limits, wherein such administration provides effective levels of the two compounds in the body of the patient. The latter also applies to cocktail therapy, e.g. the administration of three or more active ingredients.

In some embodiments, crystalline Compound 1 is incorporated into pharmaceutical compositions to provide solid oral dosage forms as defined by the claims.

In some embodiments, the diluent is selected from the group consisting of lactose, sucrose (e.g., Dipac^{®}), dextrose, dextrates, maltodextrin, mannitol, xylitol (e.g., Xylitab^{®}), sorbitol, cyclodextrins, calcium phosphate, calcium sulfate, starches, modified starches, cellulose, microcrystalline cellulose (e.g., Avicel^{®}), microcellulose, and talc.

In an aspect, a high-load formulation of ibrutinib may be advantageous as it would allow administration of one tablet per dose. Currently ibrutinib may be used in the clinic at a dose of 420 mg or 560 mg (which may be administered orally in three or four capsules comprising 140 mg ibrutinib per capsule), and hence high-load tablet formulations would be beneficial. However, high-load tablet formulations that meet pharmaceutically acceptable properties such as suitable compressibility, compactibility, granulate flowability, granulate density, integrity during manufacture, shipping and storage, proper hardness, stability, swallowability and disintegration properties when administered, are considerately more difficult to prepare than capsule formations due to the limited quantity/amount of excipients that can be used to adjust the tablet properties. Further, tablet formulations tend to have lower Cₘₐₓ as compared with the capsule formulations due to the process of its disintegration and absorption after administration, especially for ibrutinib which has a very low water solubility. It is challenging to prepare high load tablet formulations of ibrutinib that possess both pharmaceutically acceptable properties and desired PK properties, such as a high, comparable or sufficient Cₘₐₓ.

Regarding swallowability, it may be an advantage of the present invention that the pharmaceutical composition (e.g. high-load pharmaceutical tablet formulation) had good swallowability (e.g. in elderly patients too), in spite of the fact that the actual active ingredient (ibrutinib) is greater (e.g. 420 mg or 560 mg, or higher, compared to the 140 mg capsule product that is subject of the current FDA approval). The reason for this may be linked to the size/dimensions of the pharmaceutical formulation (e.g. high-load tablet), which may be comparable (or favourable) when compared with the known 140 mg capsule product. For instance, in an aspect, the tablet formulation may be of a certain dimension. When considering dimensions, the capsule that is currently approved at the US FDA has a length of about 21.7 mm, and a thickness of about 7.6 mm. The thickness of the capsule is uniform given its cylindrical shape. However, with tablets a width and thickness is given, in view of the non-cylindrical shape. Rather, the shape of the tablet is an oblong or an elongated rectangle (or even an oval shape, or a circle if the dimensions allow, e.g. if the circumference is less than 15 mm, for instance less than 10 mm), thus having the following dimensions:
a length (being the largest dimension; which is the measurement of longest distance between one end of the oblong/elongated rectangle surface to the other, provided that the distance is parallel with the longest straight edges of said oblong/elongated rectangle surface; it may also be referred to as the longest distance along the longitudinal axis);
a width (which is the measurement of the largest distance perpendicular to the length of the oblong/rectangle surface, and in the same plane as said surface); and a thickness (which is akin to the "depth" of the tablet, and is the largest distance from the top end to the bottom end of the tablet, perpendicular to the length and the width, and extending out of the plan of the oblong/elongated rectangle surface).

Thus, for the purposes herein (and unless specified otherwise), oblong encompasses an elongated rectangle shape, an oval and (when the length/width are substantially the same) a circle. However, in some embodiments, e.g. for formulations comprising greater than a 140 mg dose of ibrutinib, in an aspect, the shape of the tablet formulation is not a circle (this may be clear, for example, when the length/width is given a different dimension in the examples described hereinafter).

In an aspect, there is provided tablet formulations as described herein and with dimensions as follows:
(i) comprising 140 mg ibrutinib and wherein the length is less than 10 mm (e.g. between 5 and 10 mm, such as between 8 and 10 mm, e.g. about 9 mm), the width is less than 10 mm (e.g. between 5 and 10 mm, such as between 8 and 10 mm, e.g. about 9 mm), and the thickness is less than 5 mm (e.g. between 3 and 5 mm, such as about 4 or about 4.5 mm); in an aspect, such an embodiment may have dimensions such that the length and width are substantially the same, so forming a circle but equally such tablet shape may be an elongated rectangle or oval;
(ii) comprising 280 mg ibrutinib, and wherein the length is less than 20 mm (e.g. between 10 and 20 mm, such as between 12 and 20 mm, e.g. about 15 mm), the width is less than 10 mm (e.g. between 5 and 10 mm, such as between 8 and 10 mm, e.g. about 7 mm), and the thickness is less than 7 mm (e.g. between 4 and 7 mm, such as about 5 or about 5.5 mm); in an aspect, such an embodiment may be an elongated rectangle or oval (but, in an aspect is not a circle);
(iii) comprising 420 mg ibrutinib, and wherein the length is less than 20 mm (e.g. between 10 and 20 mm, such as between 15 and 20 mm, e.g. about 17 or 17.5 mm), the width is less than 10 mm (e.g. between 5 and 10 mm, such as between 8 and 10 mm, e.g. about 7 or 7.5 mm), and the thickness is less than 8 mm (e.g. between 4 and 8 mm, such as about 6 or about 6.5 mm); in an aspect, such an embodiment may be an elongated rectangle or oval (but, in an aspect is not a circle);
(iv)wherein the length is less than 20 or 21 mm (e.g. between 12 and 21 mm, such as between 14 and 21 mm or between 16 and 20 mm, e.g. about 19 mm), the width is less than 10 mm (e.g. between 6 and 10 mm, such as between 7 and 9 mm, e.g. about 8 mm), and the thickness is less than 9 mm (e.g. between 5 and 9 mm, such as about 7 or about 7.5 mm); in an aspect, formulations with such dimensions comprise 560 mg ibrutinib; in an aspect, such embodiments may be elongated rectangles or ovals (but, in an aspect are not circles);
(v) wherein the length is less than 25 mm (e.g. between 12 and 25 mm, such as between 14 and 25 mm or between 16 and 24 mm or between 18 and 23 mm, e.g. about 19 mm, about 21 mm or about 22 mm), the width is less than 12 mm (e.g. between 7 and 12 mm, such as between 8 and 11 mm, e.g. about 8 mm, about 9 mm or about 10 mm), and the thickness is less than 9 mm (e.g. between 5 and 9 mm, such as about 7 or about 7.5 mm); in an aspect, formulations with such dimensions comprise either 560 mg ibrutinib, 700 mg ibrutinib or 840 mg ibrutinib; in an aspect, such embodiments may be elongated rectangles or ovals (but, in an aspect are not circles).

Specific tablet formulations with dimensions may be described herein (e.g. in the examples hereinafter).

Given the overall tablet weight, particularly for the high-load doses, it is an advantage is terms of swallowability that the tablet has relatively small or favourable dimensions/size.

In an aspect, the total weight of a tablet is in an amount of about 800 mg (e.g. for the 560 mg ibrutinib dose). In other aspects, the total core weight of the tablet (without the coating) may be: between about 450 and 500 mg (e.g. for a 280 mg ibrutinib dose); between about 650 and 750 mg (e.g. for a 420 mg ibrutinib dose): between about 900 and 1000 mg (e.g. for a 560 mg ibrutinib dose); and/or between about 1300 and 1500 mg (e.g. for a 840 mg ibrutinib dose).

It is an object of the invention to provide formulations with an adequate bioavailablity (e.g. a favourable bioavailability compared to the capsule already approved by the FDA). Hence, in an aspect, there is provided a formulation in which:
- the GMR (geometric mean ratio) ranges from 75% to 92% (e.g. 80 to 85%) for Cₘₐₓ;
- the GMR for AUCₗₐₛₜ ranges from 85% to 110% (e.g. from 85 to 100%, or 85 to 95%); and/or
- the GMR for AUCᵢₙₜ (or AUC_{∞}) ranges from 80% to 105% (e.g. from 85 to 95%).

Such features relating to exposure may be a part of any of the embodiments disclosed herein.

In some embodiments, the disintegrating agent is selected from the group consisting of natural starch, a pregelatinized starch, a sodium starch, methyl crystalline cellulose, methylcellulose (e.g., Methocel^{®}), croscarmellose, croscarmellose sodium, cross-linked sodium carboxymethylcellulose, cross-linked carboxymethylcellulose, cross-linked croscarmellose, cross-linked starch such as sodium starch glycolate, cross-linked polymer such as crospovidone, cross-linked polyvinylpyrrolidone, sodium alginate, a clay, and a gum.

In some embodiments, the binder is polyvinylpyrrolidone (e.g., PVP K15, PVP K19, PVP K25, PVP K30, Povidone^{®} CL, Kollidon^{®} CL, Polyplasdone^{®} XL-10, and Povidone^{®} K-12).

In some embodiments, the surfactant is sodium lauryl sulfate.

In some embodiments, the lubricant is magnesium stearate.

Moreover, the pharmaceutical compositions described herein, which include Compound 1 as defined in the claims can be formulated into any suitable dosage form as defined in the claims.

In some embodiments, the solid dosage forms disclosed herein may be in the form of a tablet, including a suspension tablet, a fast-melt tablet, a bite-disintegration tablet, a rapid-disintegration tablet, an effervescent tablet, or a caplet, provided that the forms are as defined in the claims.In some embodiments, the pharmaceutical formulation is administered in two, or three, or four, tablets.

In some embodiments, the compositions described herein are prepared by mixing particles of Compound 1 with one or more pharmaceutical excipients to form a bulk blend composition. When referring to these bulk blend compositions as homogeneous, it is meant that the particles of Compound 1 are dispersed evenly throughout the composition so that the composition may be readily subdivided into equally effective unit dosage forms, such as tablets, pills, and capsules. The individual unit dosages may also include film coatings, which disintegrate upon oral ingestion or upon contact with diluent.

The pharmaceutical compositions or formulations described herein can further include a flavoring agent, sweetening agent, colorant, antioxidant, preservative, or one or more combination thereof. In still other aspects, using standard coating procedures, such as those described in Remington's Pharmaceutical Sciences, 20th Edition (2000), a film coating is provided around the formulation of Compound 1. In one embodiment, some or all of the particles of the Compound 1 are coated. In another embodiment, some or all of the particles of the Compound 1 are microencapsulated. In still another embodiment, the particles of the Compound 1 are not microencapsulated and are uncoated.

Suitable antioxidants for use in the compositions or formulations described herein include, for example, e.g., butylated hydroxytoluene (BHT), sodium ascorbate, and tocopherol.

It should be appreciated that there is considerable overlap between additives used in the solid dosage forms described herein. Thus, the above-listed additives should be taken as merely exemplary, and not limiting, of the types of additives that can be included in the compositions or formulations described herein. The amounts of such additives can be readily determined by one skilled in the art, according to the particular properties desired.

Compressed tablets are solid dosage forms prepared by compacting the bulk blend of the formulations described above. In various embodiments, compressed tablets which are designed to dissolve in the mouth will include one or more flavoring agents. In other embodiments, the compressed tablets will include a film surrounding the final compressed tablet. In some embodiments, the film coating can provide a delayed release of Compound 1 from the formulation. In other embodiments, the film coating aids in patient compliance (e.g., Opadry^{®} coatings or sugar coating). Film coatings including Opadry^{®} typically range from about 1% to about 3% of the tablet weight. In other embodiments, the compressed tablets include one or more excipients.

In some embodiments, the compositions or formulations described herein can be formulated as enteric coated delayed release oral dosage forms, i.e., as an oral dosage form of a pharmaceutical composition as described herein which utilizes an enteric coating to affect release in the small intestine of the gastrointestinal tract. The enteric coated dosage form may be a compressed or molded or extruded tablet/mold (coated or uncoated) containing granules, powder, pellets, beads or particles of the active ingredient and/or other composition components, which are themselves coated or uncoated. The enteric coated oral dosage form may also be a capsule (coated or uncoated) containing pellets, beads or granules of the solid carrier or the composition, which are themselves coated or uncoated.

The term "delayed release" as used herein refers to the delivery so that the release can be accomplished at some generally predictable location in the intestinal tract more distal to that which would have been accomplished if there had been no delayed release alterations. In some embodiments the method for delay of release is coating. Any coatings should be applied to a sufficient thickness such that the entire coating does not dissolve in the gastrointestinal fluids at pH below about 5, but does dissolve at pH about 5 and above. It is expected that any anionic polymer exhibiting a pH-dependent solubility profile can be used as an enteric coating in the methods and compositions described herein to achieve delivery to the lower gastrointestinal tract. In some embodiments the polymers described herein are anionic carboxylic polymers. In other embodiments, the polymers and compatible mixtures thereof, and some of their properties, include, but are not limited to:
Shellac, also called purified lac, a refined product obtained from the resinous secretion of an insect. This coating dissolves in media of pH >7;
Acrylic polymers. The performance of acrylic polymers (primarily their solubility in biological fluids) can vary based on the degree and type of substitution. Examples of suitable acrylic polymers include methacrylic acid copolymers and ammonium methacrylate copolymers. The Eudragit series E, L, S, RL, RS and NE (Rohm Pharma) are available as solubilized in organic solvent, aqueous dispersion, or dry powders. The Eudragit series RL, NE, and RS are insoluble in the gastrointestinal tract but are permeable and are used primarily for colonic targeting. The Eudragit series E dissolve in the stomach. The Eudragit series L, L-30D and S are insoluble in stomach and dissolve in the intestine;
Cellulose Derivatives. Examples of suitable cellulose derivatives are: ethyl cellulose; reaction mixtures of partial acetate esters of cellulose with phthalic anhydride. The performance can vary based on the degree and type of substitution. Cellulose acetate phthalate (CAP) dissolves in pH >6. Aquateric (FMC) is an aqueous based system and is a spray dried CAP psuedolatex with particles <1 µm. Other components in Aquateric can include pluronics, Tweens, and acetylated monoglycerides. Other suitable cellulose derivatives include: cellulose acetate trimellitate (Eastman); methylcellulose (Pharmacoat, Methocel); hydroxypropylmethyl cellulose phthalate (HPMCP); hydroxypropylmethyl cellulose succinate (HPMCS); and
hydroxypropylmethylcellulose acetate succinate (e.g., AQOAT (Shin Etsu)). The performance can vary based on the degree and type of substitution. For example, HPMCP such as, HP-50, HP-55, HP-55S, HP-55F grades are suitable. The performance can vary based on the degree and type of substitution. For example, suitable grades of hydroxypropylmethylcellulose acetate succinate include, but are not limited to, AS-LG (LF), which dissolves at pH 5, AS-MG (MF), which dissolves at pH 5.5, and AS-HG (HF), which dissolves at higher pH. These polymers are offered as granules, or as fine powders for aqueous dispersions; Poly Vinyl Acetate Phthalate (PVAP). PVAP dissolves in pH >5, and it is much less permeable to water vapor and gastric fluids.

In some embodiments, the coating can, and usually does, contain a plasticizer and possibly other coating excipients such as colorants, talc, and/or magnesium stearate, which are well known in the art. Suitable plasticizers include triethyl citrate (Citroflex 2), triacetin (glyceryl triacetate), acetyl triethyl citrate (Citroflec A2), Carbowax 400 (polyethylene glycol 400), diethyl phthalate, tributyl citrate, acetylated monoglycerides, glycerol, fatty acid esters, propylene glycol, and dibutyl phthalate. In particular, anionic carboxylic acrylic polymers usually will contain 10-25% by weight of a plasticizer, especially dibutyl phthalate, polyethylene glycol, triethyl citrate and triacetin. Conventional coating techniques such as spray or pan coating are employed to apply coatings. The coating thickness must be sufficient to ensure that the oral dosage form remains intact until the desired site of topical delivery in the intestinal tract is reached.

Colorants, detackifiers, surfactants, antifoaming agents, lubricants (e.g., carnuba wax or PEG) may be added to the coatings besides plasticizers to solubilize or disperse the coating material, and to improve coating performance and the coated product.

In other embodiments, the formulations described herein, which include Compound 1, are delivered using a pulsatile dosage form. A pulsatile dosage form is capable of providing one or more immediate release pulses at predetermined time points after a controlled lag time or at specific sites. Many other types of controlled release systems known to those of ordinary skill in the art and are suitable for use with the formulations described herein. Examples of such delivery systems include, e.g., polymer-based systems, such as polylactic and polyglycolic acid, plyanhydrides and polycaprolactone; porous matrices, nonpolymer-based systems that are lipids, including sterols, such as cholesterol, cholesterol esters and fatty acids, or neutral fats, such as mono-, di- and triglycerides; hydrogel release systems; silastic systems; peptide-based systems; wax coatings, bioerodible dosage forms, compressed tablets using conventional binders and the like. See, e.g., Liberman et al., Pharmaceutical Dosage Forms, 2 Ed., Vol. 1, pp. 209-214 (1990); Singh et al., Encyclopedia of Pharmaceutical Technology, 2nd Ed., pp. 751-753 (2002); U.S. Pat. Nos. 4,327,725, 4,624,848, 4,968,509, 5,461,140, 5,456,923, 5,516,527, 5,622,721, 5,686,105, 5,700,410, 5,977,175, 6,465,014 and 6,932,983.

It is to be appreciated that there is overlap between the above-listed additives used in the aqueous dispersions or suspensions described herein, since a given additive is often classified differently by different practitioners in the field, or is commonly used for any of several different functions. Thus, the above-listed additives should be taken as merely exemplary, and not limiting, of the types of additives that can be included in formulations described herein. The amounts of such additives can be readily determined by one skilled in the art, according to the particular properties desired.

### Dosing and Treatment Regimens

In some embodiments, the amount of Compound 1 that is administered to a mammal is from 300 mg/day up to, and including, 1000 mg/day. In some embodiments, the amount of Compound 1 that is administered to a mammal is from 420 mg/day up to, and including, 840 mg/day. In some embodiments, the amount of Compound 1 that is administered to a mammal is about 420 mg/day, about 560 mg/day, or about 840 mg/day. In some embodiments, the amount of Compound 1 that is administered to a mammal is about 420 mg/day. In some embodiments, the amount of Compound 1 that is administered to a mammal is about 560 mg/day. In some embodiments, the AUC₀₋₂₄ of Compound 1 is between about 150 and about 3500 ng*h/mL. In some embodiments, the AUC₀₋₂₄ of Compound 1 is between about 500 and about 1100 ng*h/mL. In some embodiments, Compound 1 is administered orally. In some embodiments, Compound 1 is administered once per day, twice per day, or three times per day. In some embodiments, Compound 1 is administered daily. In some embodiments, Compound 1 is administered once daily. In some embodiments, Compound 1 is administered every other day. In some embodiments, the Compound 1 is a maintenance therapy.

Compound 1 can be used in the preparation of medicaments for the inhibition of Btk or a homolog thereof, or for the treatment of diseases or conditions that would benefit, at least in part, from inhibition of Btk or a homolog thereof, including a subject diagnosed with a hematological malignancy. In addition, a method for treating any of the diseases or conditions described herein in a subject in need of such treatment, involves administration of pharmaceutical compositions containing Compound 1, or a pharmaceutically acceptable salt, pharmaceutically acceptable N-oxide, pharmaceutically active metabolite, pharmaceutically acceptable prodrug, or pharmaceutically acceptable solvate thereof, in therapeutically effective amounts to said subject.

The compositions containing Compound 1 can be administered for prophylactic, therapeutic, or maintenance treatment. In some embodiments, compositions containing Compound 1 are administered for therapeutic applications (e.g., administered to a subject diagnosed with a hematological malignancy). In some embodiments, compositions containing Compound 1 are administered for therapeutic applications (e.g., administered to a subject susceptible to or otherwise at risk of developing a hematological malignancy). In some embodiments, compositions containing Compound 1 are administered to a patient who is in remission as a maintenance therapy.

Amounts of Compound 1 will depend on the use (e.g., therapeutic, prophylactic, or maintenance). Amounts of Compound 1 will depend on severity and course of the disease or condition, previous therapy, the patient's health status, weight, and response to the drugs, and the judgment of the treating physician. It is considered well within the skill of the art for one to determine such therapeutically effective amounts by routine experimentation (including, but not limited to, a dose escalation clinical trial). In some embodiments, the amount of Compound 1 is from 300 mg/day up to, and including, 1000 mg/day. In some embodiments, the amount of Compound 1 is from 420 mg/day up to, and including, 840 mg/day. In some embodiments, the amount of Compound 1 is from 400 mg/day up to, and including, 860 mg/day. In some embodiments, the amount of Compound 1 is about 360 mg/day. In some embodiments, the amount of Compound 1 is about 420 mg/day. In some embodiments, the amount of Compound 1 is about 560 mg/day. In some embodiments, the amount of Compound 1 is about 840 mg/day. In some embodiments, the amount of Compound 1 is from 2 mg/kg/day up to, and including, 13 mg/kg/day. In some embodiments, the amount of Compound 1 is from 2.5 mg/kg/day up to, and including, 8 mg/kg/day. In some embodiments, the amount of Compound 1 is from 2.5 mg/kg/day up to, and including, 6 mg/kg/day. In some embodiments, the amount of Compound 1 is from 2.5 mg/kg/day up to, and including, 4 mg/kg/day. In some embodiments, the amount of Compound 1 is about 2.5 mg/kg/day. In some embodiments, the amount of Compound 1 is about 8 mg/kg/day.

In some embodiments, pharmaceutical compositions described herein include about 140 mg of Compound 1. In some embodiments, a tablet formulation is prepared that includes about 140 mg of Compound 1. In some embodiments, 2, 3, 4, or 5 of the tablet formulations are administered daily. In some embodiments, 3 or 4 of the capsules are administered daily. In some embodiments tablet are administered once daily. In some embodiments, the capsules are administered once daily. In other embodiments, the tablet are administered multiple times a day.

In some embodiments, Compound 1 is administered daily. In some embodiments, Compound 1 is administered every other day.

In some embodiments, Compound 1 is administered once per day. In some embodiments, Compound 1 is administered twice per day. In some embodiments, Compound 1 is administered three times per day. In some embodiments, Compound 1 is administered four times per day.

In some embodiments, Compound 1 is administered until disease progression, unacceptable toxicity, or individual choice. In some embodiments, Compound 1 is administered daily until disease progression, unacceptable toxicity, or individual choice. In some embodiments, Compound 1 is administered every other day until disease progression, unacceptable toxicity, or individual choice.

In the case wherein the patient's status does improve, upon the doctor's discretion the administration of the compounds may be given continuously; alternatively, the dose of drug being administered may be temporarily reduced or temporarily suspended for a certain length of time (i.e., a "drug holiday"). The length of the drug holiday can vary between 2 days and 1 year, including by way of example only, 2 days, 3 days, 4 days, 5 days, 6 days, 7 days, 10 days, 12 days, 15 days, 20 days, 28 days, 35 days, 50 days, 70 days, 100 days, 120 days, 150 days, 180 days, 200 days, 250 days, 280 days, 300 days, 320 days, 350 days, or 365 days. The dose reduction during a drug holiday may be from 10%-100%, including, by way of example only, 10%, 15%, 20%, 25%, 30%, 35%, 40%, 45%, 50%, 55%, 60%, 65%, 70%, 75%, 80%, 85%, 90%, 95%, or 100%.

Once improvement of the patient's conditions has occurred, a maintenance dose is administered if necessary. Subsequently, the dosage or the frequency of administration, or both, can be reduced, as a function of the symptoms, to a level at which the improved disease, disorder or condition is retained. Patients can, however, require intermittent treatment on a long-term basis upon any recurrence of symptoms.

The amount of a given agent that will correspond to such an amount will vary depending upon factors such as the particular compound, the severity of the disease, the identity (e.g., weight) of the subject or host in need of treatment, but can nevertheless be routinely determined in a manner known in the art according to the particular circumstances surrounding the case, including, e.g., the specific agent being administered, the route of administration, and the subject or host being treated. In general, however, doses employed for adult human treatment will typically be in the range of 0.02-5000 mg per day, or from about 1-1500 mg per day. The desired dose may conveniently be presented in a single dose or as divided doses administered simultaneously (or over a short period of time) or at appropriate intervals, for example as two, three, four or more sub-doses per day.

The pharmaceutical compositions or formulations described herein may be in unit dosage forms suitable for single administration of precise dosages. In unit dosage form, the formulation is divided into unit doses containing appropriate quantities of one or more compound. The unit dosage may be in the form of a package containing discrete quantities of the formulation. Non-limiting examples are packaged tablets or capsules, and powders in vials or ampoules. Aqueous suspension compositions can be packaged in single-dose non-reclosable containers. Alternatively, multiple-dose reclosable containers can be used, in which case it is typical to include a preservative in the composition. In some embodiments, each unit dosage form comprises 140 mg of Compound 1. In some embodiments, an individual is administered 1 unit dosage form per day. In some embodiments, an individual is administered 2 unit dosage forms per day. In some embodiments, an individual is administered 3 unit dosage forms per day. In some embodiments, an individual is administered 4 unit dosage forms per day.

The foregoing ranges are merely suggestive, as the number of variables in regard to an individual treatment regime is large, and considerable excursions from these recommended values are not uncommon. Such dosages may be altered depending on a number of variables, not limited to the activity of the compound used, the disease or condition to be treated, the mode of administration, the requirements of the individual subject, the severity of the disease or condition being treated, and the judgment of the practitioner.

Toxicity and therapeutic efficacy of such therapeutic regimens can be determined by standard pharmaceutical procedures in cell cultures or experimental animals, including, but not limited to, the determination of the LD₅₀ (the dose lethal to 50% of the population) and the ED₅₀ (the dose therapeutically effective in 50% of the population). The dose ratio between the toxic and therapeutic effects is the therapeutic index and it can be expressed as the ratio between LD₅₀ and ED₅₀. Compounds exhibiting high therapeutic indices are preferred. The data obtained from cell culture assays and animal studies can be used in formulating a range of dosage for use in human. The dosage of such compounds lies preferably within a range of circulating concentrations that include the ED₅₀ with minimal toxicity. The dosage may vary within this range depending upon the dosage form employed and the route of administration utilized.

### Combination Therapy

In certain instances, it is appropriate to administer Compound 1 in combination with another therapeutic agent.

In one embodiment, the compositions and methods described herein are also used in conjunction with other therapeutic reagents that are selected for their particular usefulness against the condition that is being treated. In general, the compositions described herein and, in embodiments where combinational therapy is employed, other agents do not have to be administered in the same pharmaceutical composition, and are, because of different physical and chemical characteristics, administered by different routes. In one embodiment, the initial administration is made according to established protocols, and then, based upon the observed effects, the dosage, modes of administration and times of administration, further modified.

In various embodiments, the compounds are administered concurrently (e.g., simultaneously, essentially simultaneously or within the same treatment protocol) or sequentially, depending upon the nature of the disease, the condition of the patient, and the actual choice of compounds used. In certain embodiments, the determination of the order of administration, and the number of repetitions of administration of each therapeutic agent during a treatment protocol, is based upon evaluation of the disease being treated and the condition of the patient.

For combination therapies described herein, dosages of the co-administered compounds vary depending on the type of co-drug employed, on the specific drug employed, on the disease or condition being treated and so forth.

The individual compounds of such combinations are administered either sequentially or simultaneously in separate or combined pharmaceutical formulations. In one embodiment, the individual compounds will be administered simultaneously in a combined pharmaceutical formulation. Appropriate doses of known therapeutic agents will be appreciated by those skilled in the art.

The combinations referred to herein are conveniently presented for use in the form of a pharmaceutical compositions together with a pharmaceutically acceptable diluent(s) or carrier(s).

Disclosed herein, in certain embodiments, is a method for treating a cancer in an individual in need thereof, comprising: administering to the individual an amount of Compound 1. In some embodiments, the method further comprises administering a second cancer treatment regimen.

In some embodiments, administering a Btk inhibitor before a second cancer treatment regimen reduces immune-mediated reactions to the second cancer treatment regimen. In some embodiments, administering Compound 1 before ofatumumab reduces immune-mediated reactions to ofatumumab.

In some embodiments, the second cancer treatment regimen comprises a chemotherapeutic agent, a steroid, an immunotherapeutic agent, a targeted therapy, or a combination thereof. In some embodiments, the second cancer treatment regimen comprises a B cell receptor pathway inhibitor. In some embodiments, the B cell receptor pathway inhibitor is a CD79A inhibitor, a CD79B inhibitor, a CD19 inhibitor, a Lyn inhibitor, a Syk inhibitor, a PI3K inhibitor, a Blnk inhibitor, a PLCγ inhibitor, a PKCβ inhibitor, or a combination thereof. In some embodiments, the second cancer treatment regimen comprises an antibody, B cell receptor signaling inhibitor, a PI3K inhibitor, an IAP inhibitor, an mTOR inhibitor, an immunochemotherapy, a radio immunotherapeutic, a DNA damaging agent, a proteosome inhibitor, a Cyp3A4 inhibitor, a histone deacetylase inhibitor, a protein kinase inhibitor, a hedgehog inhibitor, an Hsp90 inhibitor, a telomerase inhibitor, a Jak1/2 inhibitor, a protease inhibitor, a PKC inhibitor, a PARP inhibitor, or a combination thereof.

In some embodiments, the second cancer treatment regimen comprises chlorambucil, ifosphamide, doxorubicin, mesalazine, thalidomide, lenalidomide, temsirolimus, everolimus, fludarabine, fostamatinib, paclitaxel, docetaxel, ofatumumab, rituximab, dexamethasone, prednisone, CAL-101, ibritumomab, tositumomab, bortezomib, pentostatin, endostatin, EPOCH-R, DA-EPOCH-R, rifampin, selinexor, gemcitabine, obinutuzumab, carmustine, cytarabine, melphalan, ublituximab, palbociclib, ACP-196 (Acerta Pharma BV), TGR-1202 (TG Therapeutics, Inc.), TEDDI, TEDD, MEDI4736 (AstraZeneca), ABT-0199 (AbbVie), CC-122 (Celgene Corporation), LD-AraC, ketoconazole, etoposide, carboplatin, moxifloxacin, citrovorum, methotrexate, filgrastim, mesna, vincristine, cyclophosphamide, erythromycin, voriconazole, nivolumab, or a combination thereof.

In some embodiments, the second cancer treatment regimen comprises cyclophosphamide, hydroxydaunorubicin, vincristine, and prednisone, and optionally, rituximab.

In some embodiments, the second cancer treatment regimen comprises bendamustine, and rituximab.

In some embodiments, the second cancer treatment regimen comprises fludarabine, cyclophosphamide, and rituximab.

In some embodiments, the second cancer treatment regimen comprises cyclophosphamide, vincristine, and prednisone, and optionally, rituximab.

In some embodiments, the second cancer treatment regimen comprises etoposide, doxorubicin, vinristine, cyclophosphamide, prednisolone, and optionally, rituximab.

In some embodiments, the second cancer treatment regimen comprises dexamethasone and lenalidomide.

In some embodiments, the second cancer treatment comprises a proteasome inhibitor. In some embodiments, the second treatment comprises bortezomib. In some embodiments, the second cancer treatment comprises an epoxyketone. In some embodiments, the second cancer treatment comprises epoxomicin. In some embodiments, the second cancer treatment comprises a tetrapeptide epoxyketone In some embodiments, the second cancer treatment comprises carfilzomib. In some embodiments, the second cancer treatment comprises disulfram, epigallocatechin-3-gallate, salinosporamide A, ONX 0912m CEP-18770, MLN9708, or MG132.

In some embodiments, the second cancer treatment comprises a Cyp3A4 inhibitor. In some embodiments, the second cancer treatment comprises indinavir, nelfinavir, ritonavir, clarithromycin, itraconazole, ketoconazole, nefazodone. In some embodiments, the second cancer treatment comprises ketoconazole.

In some embodiments, the second cancer treatment comprises a Janus Kinase (JAK) inhibitor. In some embodiments, the second treatment comprises Lestaurtinib, Tofacitinib, Ruxolitinib, CYT387, Baricitinib or Pacritinib.

In some embodiments, the second cancer treatment comprises a histone deacetylase inhibitor (HDAC inhibitor, HDI). In some embodiments, the second cancer treatment comprises a hydroxamic acid (or hydroxamate), such as trichostatin A, vorinostat (SAHA), belinostat (PXD101), LAQ824, and panobinostat (LBH589), a cyclic tetrapeptide, such as trapoxin B, a depsipeptide, a benzamide, such as entinostat (MS-275), CI994, and mocetinostat (MGCD0103), an electrophilic ketone, or an aliphatic acid compound, such as phenylbutyrate and valproic acid,

Additional cancer treatment regimens include Nitrogen Mustards such as for example, bendamustine, chlorambucil, chlormethine, cyclophosphamide, ifosfamide, melphalan, prednimustine, trofosfamide; Alkyl Sulfonates like busulfan, mannosulfan, treosulfan; Ethylene Imines like carboquone, thiotepa, triaziquone; Nitrosoureas like carmustine, fotemustine, lomustine, nimustine, ranimustine, semustine, streptozocin; Epoxides such as for example, etoglucid; Other Alkylating Agents such as for example dacarbazine, mitobronitol, pipobroman, temozolomide; Folic Acid Analogues such as for example methotrexate, permetrexed, pralatrexate, raltitrexed; Purine Analogs such as for example cladribine, clofarabine, fludarabine, mercaptopurine, nelarabine, tioguanine; Pyrimidine Analogs such as for example azacitidine, capecitabine, carmofur, cytarabine, decitabine, fluorouracil, gemcitabine, tegafur; Vinca Alkaloids such as for example vinblastine, vincristine, vindesine, vinflunine, vinorelbine; Podophyllotoxin Derivatives such as for example etoposide, teniposide; Colchicine derivatives such as for example demecolcine; Taxanes such as for example docetaxel, paclitaxel, paclitaxel poliglumex; Other Plant Alkaloids and Natural Products such as for example trabectedin; Actinomycines such as for example dactinomycin; Antracyclines such as for example aclarubicin, daunorubicin, doxorubicin, epirubicin, idarubicin, mitoxantrone, pirarubicin, valrubicin, zorubincin; Other Cytotoxic Antibiotics such as for example bleomycin, ixabepilone, mitomycin, plicamycin; Platinum Compounds such as for example carboplatin, cisplatin, oxaliplatin, satraplatin; Methylhydrazines such as for example procarbazine; Sensitizers such as for example aminolevulinic acid, efaproxiral, methyl aminolevulinate, porfimer sodium, temoporfin; Protein Kinase Inhibitors such as for example dasatinib, erlotinib, everolimus, gefitinib, imatinib, lapatinib, nilotinib, pazonanib, sorafenib, sunitinib, temsirolimus; Other Antineoplastic Agents such as for example alitretinoin, altretamine, amzacrine, anagrelide, arsenic trioxide, asparaginase, bexarotene, bortezomib, celecoxib, denileukin diftitox, estramustine, hydroxycarbamide, irinotecan, lonidamine, masoprocol, miltefosein, mitoguazone, mitotane, oblimersen, pegaspargase, pentostatin, romidepsin, sitimagene ceradenovec, tiazofurine, topotecan, tretinoin, vorinostat; Estrogens such as for example diethylstilbenol, ethinylestradiol, fosfestrol, polyestradiol phosphate; Progestogens such as for example gestonorone, medroxyprogesterone, megestrol; Gonadotropin Releasing Hormone Analogs such as for example buserelin, goserelin, leuprorelin, triptorelin; Anti-Estrogens such as for example fulvestrant, tamoxifen, toremifene; Anti-Androgens such as for example bicalutamide, flutamide, nilutamide; Enzyme Inhibitors, aminoglutethimide, anastrozole, exemestane, formestane, letrozole, vorozole; Other Hormone Antagonists such as for example abarelix, degarelix; Immunostimulants such as for example histamine dihydrochloride, mifamurtide, pidotimod, plerixafor, roquinimex, thymopentin; Immunosuppressants such as for example everolimus, gusperimus, leflunomide, mycophenolic acid, sirolimus; Calcineurin Inhibitors such as for example ciclosporin, tacrolimus; Other Immunosuppressants such as for example azathioprine, lenalidomide, methotrexate, thalidomide; and Radiopharmaceuticals such as for example, iobenguane.

Additional cancer treatment regimens include interferons, interleukins, Tumor Necrosis Factors, Growth Factors, or the like.

Additional cancer treatment regimens include Immunostimulants such as for example ancestim, filgrastim, lenograstim, molgramostim, pegfilgrastim, sargramostim; Interferons such as for example interferon alfa natural, interferon alfa-2a, interferon alfa-2b, interferon alfacon-1, interferon alfa-n1, interferon beta natural, interferon beta-1a, interferon beta-1b, interferon gamma, peginterferon alfa-2a, peginterferon alfa-2b; Interleukins such as for example aldesleukin, oprelvekin; Other Immunostimulants such as for example BCG vaccine, glatiramer acetate, histamine dihydrochloride, immunocyanin, lentinan, melanoma vaccine, mifamurtide, pegademase, pidotimod, plerixafor, poly I:C, poly ICLC, roquinimex, tasonermin, thymopentin; Immunosuppressants such as for example abatacept, abetimus, alefacept, antilymphocyte immunoglobulin (horse), antithymocyte immunoglobulin (rabbit), eculizumab, efalizumab, everolimus, gusperimus, leflunomide, muromab-CD3, mycophenolic acid, natalizumab, sirolimus; TNF alpha Inhibitors such as for example adalimumab, afelimomab, certolizumab pegol, etanercept, golimumab, infliximab; Interleukin Inhibitors such as for example anakinra, basiliximab, canakinumab, daclizumab, mepolizumab, rilonacept, tocilizumab, ustekinumab; Calcineurin Inhibitors such as for example ciclosporin, tacrolimus; Other Immunosuppressants such as for example azathioprine, lenalidomide, methotrexate, thalidomide.

Additional cancer treatment regimens include Adalimumab, Alemtuzumab, Basiliximab, Bevacizumab, Cetuximab, Certolizumab pegol, Daclizumab, Eculizumab, Efalizumab, Gemtuzumab, Ibritumomab tiuxetan, Infliximab, Muromonab-CD3, Natalizumab, Panitumumab, Ranibizumab, Rituximab, Tositumomab, Trastuzumab, or the like, or a combination thereof.

Additional cancer treatment regimens include Monoclonal Antibodies such as for example alemtuzumab, bevacizumab, catumaxomab, cetuximab, edrecolomab, gemtuzumab, ofatumumab, panitumumab, rituximab, trastuzumab, , Immunosuppressants, eculizumab, efalizumab, muromab-CD3, natalizumab; TNF alpha Inhibitors such as for example adalimumab, afelimomab, certolizumab pegol, golimumab, infliximab, , Interleukin Inhibitors, basiliximab, canakinumab, daclizumab, mepolizumab, tocilizumab, ustekinumab, , Radiopharmaceuticals, ibritumomab tiuxetan, tositumomab; Others Monoclonal Antibodies such as for example abagovomab, adecatumumab, alemtuzumab, anti-CD30 monoclonal antibody Xmab2513, anti-MET monoclonal antibody MetMab, apolizumab, apomab, arcitumomab, basiliximab, bispecific antibody 2B1, blinatumomab, brentuximab vedotin, capromab pendetide, cixutumumab, claudiximab, conatumumab, dacetuzumab, denosumab, eculizumab, epratuzumab, epratuzumab, ertumaxomab, etaracizumab, figitumumab, fresolimumab, galiximab, ganitumab, gemtuzumab ozogamicin, glembatumumab, ibritumomab, inotuzumab ozogamicin, ipilimumab, lexatumumab, lintuzumab, lintuzumab, lucatumumab, mapatumumab, matuzumab, milatuzumab, monoclonal antibody CC49, necitumumab, nimotuzumab, ofatumumab, oregovomab, pertuzumab, ramacurimab, ranibizumab, siplizumab, sonepcizumab, tanezumab, tositumomab, trastuzumab, tremelimumab, tucotuzumab celmoleukin, veltuzumab, visilizumab, volociximab, zalutumumab.

Additional cancer treatment regimens include agents that affect the tumor micro-environment such as cellular signaling network (e.g. phosphatidylinositol 3-kinase (PI3K) signaling pathway, signaling from the B-cell receptor and the IgE receptor). In some embodiments, the second agent is a PI3K signaling inhibitor or a syc kinase inhibitor. In one embodiment, the syk inhibitor is R788. In another embodiment is a PKCy inhibitor such as by way of example only, enzastaurin.

Examples of agents that affect the tumor micro-environment include PI3K signaling inhibitor, syc kinase inhibitor, Protein Kinase Inhibitors such as for example dasatinib, erlotinib, everolimus, gefitinib, imatinib, lapatinib, nilotinib, pazonanib, sorafenib, sunitinib, temsirolimus; Other Angiogenesis Inhibitors such as for example GT-111, JI-101, R1530; Other Kinase Inhibitors such as for example AC220, AC480, ACE-041, AMG 900, AP24534, Arry-614, AT7519, AT9283, AV-951, axitinib, AZD1152, AZD7762, AZD8055, AZD8931, bafetinib, BAY 73-4506, BGJ398, BGT226, BI 811283, BI6727, BIBF 1120, BIBW 2992, BMS-690154, BMS-777607, BMS-863233, BSK-461364, CAL-101, CEP-11981, CYC116, DCC-2036, dinaciclib, dovitinib lactate, E7050, EMD 1214063, ENMD-2076, fostamatinib disodium, GSK2256098, GSK690693, INCB18424, INNO-406, JNJ-26483327, JX-594, KX2-391, linifanib, LY2603618, MGCD265, MK-0457, MK1496, MLN8054, MLN8237, MP470, NMS-1116354, NMS-1286937, ON 01919.Na, OSI-027, OSI-930, Btk inhibitor, PF-00562271, PF-02341066, PF-03814735, PF-04217903, PF-04554878, PF-04691502, PF-3758309, PHA-739358, PLC3397, progenipoietin, R547, R763, ramucirumab, regorafenib, RO5185426, SAR103168, SCH 727965, SGI-1176, SGX523, SNS-314, TAK-593, TAK-901, TKI258, TLN-232, TTP607, XL147, XL228, XL281R05126766, XL418, XL765.

Further examples of anti-cancer agents for use in combination with a Btk inhibitor compound include inhibitors of mitogen-activated protein kinase signaling, e.g., U0126, PD98059, PD184352, PD0325901, ARRY-142886, SB239063, SP600125, BAY 43-9006, wortmannin, or LY294002; Syk inhibitors; mTOR inhibitors; and antibodies (e.g., rituxan).

Other anti-cancer agents that can be employed in combination with a Btk inhibitor compound include Adriamycin, Dactinomycin, Bleomycin, Vinblastine, Cisplatin, acivicin; aclarubicin; acodazole hydrochloride; acronine; adozelesin; aldesleukin; altretamine; ambomycin; ametantrone acetate; aminoglutethimide; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; chlorambucil; cirolemycin; cladribine; crisnatol mesylate; cyclophosphamide; cytarabine; dacarbazine; daunorubicin hydrochloride; decitabine; dexormaplatin; dezaguanine; dezaguanine mesylate; diaziquone; doxorubicin; doxorubicin hydrochloride; droloxifene; droloxifene citrate; dromostanolone propionate; duazomycin; edatrexate; eflornithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone; fostriecin sodium; gemcitabine; gemcitabine hydrochloride; hydroxyurea; idarubicin hydrochloride; ifosfamide; iimofosine; interleukin Il (including recombinant interleukin II, or rlL2), interferon alfa-2a; interferon alfa-2b; interferon alfa-n1; interferon alfa-n3; interferon beta-1a; interferon gamma-1 b; iproplatin; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazoie; nogalamycin; ormaplatin; oxisuran; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; zorubicin hydrochloride.

Other anti-cancer agents that can be employed in combination with a Btk inhibitor compound include: 20-epi-1, 25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; 9- dioxamycin; diphenyl spiromustine; docosanol; dolasetron; doxifluridine; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-such as for example growth factor-1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; lovastatin; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A+myobacterium cell wall sk; mopidamol; multiple drug resistance gene inhibitor; multiple tumor suppressor 1 -based therapy; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; O6-benzylguanine; octreotide; okicenone; oligonucleotides; onapristone; ondansetron; ondansetron; oracin; oral cytokine inducer; ormaplatin; osaterone; oxaliplatin; oxaunomycin; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylerie conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen-binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; velaresol; veramine; verdins; verteporfin; vinorelbine; vinxaltine; vitaxin; vorozole; zanoterone; zeniplatin; zilascorb; and zinostatin stimalamer.

Yet other anticancer agents that can be employed in combination with a Btk inhibitor compound include alkylating agents, antimetabolites, natural products, or hormones, e.g., nitrogen mustards (e.g., mechloroethamine, cyclophosphamide, chlorambucil, etc.), alkyl sulfonates (e.g., busulfan), nitrosoureas (e.g., carmustine, lomusitne, ete.), or triazenes (decarbazine, etc.). Examples of antimetabolites include but are not limited to folic acid analog (e.g., methotrexate), or pyrimidine analogs (e.g., Cytarabine), purine analogs (e.g., mercaptopurine, thioguanine, pentostatin).

Examples of alkylating agents that can be employed in combination a Btk inhibitor compound include, but are not limited to, nitrogen mustards (e.g., mechloroethamine, cyclophosphamide, chlorambucil, meiphalan, etc.), ethylenimine and methylmelamines (e.g., hexamethlymelamine, thiotepa), alkyl sulfonates (e.g., busulfan), nitrosoureas (e.g., carmustine, lomusitne, semustine, streptozocin, etc.), or triazenes (decarbazine, ete.). Examples of antimetabolites include, but are not limited to folic acid analog (e.g., methotrexate), or pyrimidine analogs (e.g., fluorouracil, floxouridine, Cytarabine), purine analogs (e.g., mercaptopurine, thioguanine, pentostatin.

Examples of anti-cancer agents which act by arresting cells in the G2-M phases due to stabilized microtubules and which can be used in combination with a Btk inhibitor compound include without limitation the following marketed drugs and drugs in development: Erbulozole (also known as R-55104), Dolastatin 10 (also known as DLS-10 and NSC-376128), Mivobulin isethionate (also known as CI-980), Vincristine, NSC-639829, Discodermolide (also known as NVP-XX-A-296), ABT-751 (Abbott, also known as E-7010), Altorhyrtins (such as Altorhyrtin A and Altorhyrtin C), Spongistatins (such as Spongistatin 1, Spongistatin 2, Spongistatin 3, Spongistatin 4, Spongistatin 5, Spongistatin 6, Spongistatin 7, Spongistatin 8, and Spongistatin 9), Cemadotin hydrochloride (also known as LU-103793 and NSC-D-669356), Epothilones (such as Epothilone A, Epothilone B, Epothilone C (also known as desoxyepothilone A or dEpoA), Epothilone D (also referred to as KOS-862, dEpoB, and desoxyepothilone B ), Epothilone E, Epothilone F, Epothilone B N-oxide, Epothilone A N-oxide, 16-aza-epothilone B, 21-aminoepothilone B (also known as BMS-310705), 21-hydroxyepothilone D (also known as Desoxyepothilone F and dEpoF), 26-fluoroepothilone), Auristatin PE (also known as NSC-654663), Soblidotin (also known as TZT-1027), LS-4559-P (Pharmacia, also known as LS-4577), LS-4578 (Pharmacia, also known as LS-477-P), LS-4477 (Pharmacia), LS-4559 (Pharmacia), RPR-112378 (Aventis), Vincristine sulfate, DZ-3358 (Daiichi), FR-182877 (Fujisawa, also known as WS-9885B), GS-164 (Takeda), GS-198 (Takeda), KAR-2 (Hungarian Academy of Sciences), BSF-223651 (BASF, also known as ILX-651 and LU-223651 ), SAH-49960 (Lilly/Novartis), SDZ-268970 (Lilly/Novartis), AM-97 (Armad/Kyowa Hakko), AM-132 (Armad), AM-138 (Armad/Kyowa Hakko), IDN-5005 (Indena), Cryptophycin 52 (also known as LY-355703), AC-7739 (Ajinomoto, also known as AVE-8063A and CS-39.HCI), AC-7700 (Ajinomoto, also known as AVE-8062, AVE-8062A, CS-39-L-Ser.HCI, and RPR-258062A), Vitilevuamide, Tubulysin A, Canadensol, Centaureidin (also known as NSC-106969), T-138067 (Tularik, also known as T-67, TL-138067 and TI-138067), COBRA-1 (Parker Hughes Institute, also known as DDE-261 and WHI-261), H10 (Kansas State University), H16 (Kansas State University), Oncocidin A1 (also known as BTO-956 and DIME), DDE-313 (Parker Hughes Institute), Fijianolide B, Laulimalide, SPA-2 (Parker Hughes Institute), SPA-1 (Parker Hughes Institute, also known as SPIKET-P), 3-IAABU (Cytoskeleton/Mt. Sinai School of Medicine, also known as MF-569), Narcosine (also known as NSC-5366), Nascapine, D-24851 (Asta Medica), A-105972 (Abbott), Hemiasterlin, 3-BAABU (Cytoskeleton/Mt. Sinai School of Medicine, also known as MF-191), TMPN (Arizona State University), Vanadocene acetylacetonate, T-138026 (Tularik), Monsatrol, lnanocine (also known as NSC-698666), 3-lAABE (Cytoskeleton/Mt. Sinai School of Medicine), A-204197 (Abbott), T-607 (Tularik, also known as T-900607), RPR- 115781 (Aventis), Eleutherobins (such as Desmethyleleutherobin, Desaetyleleutherobin, lsoeleutherobin A, and Z-Eleutherobin), Caribaeoside, Caribaeolin, Halichondrin B, D-64131 (Asta Medica), D-68144 (Asta Medica), Diazonamide A, A-293620 (Abbott), NPI-2350 (Nereus), Taccalonolide A, TUB-245 (Aventis), A-259754 (Abbott), Diozostatin, (-)-Phenylahistin (also known as NSCL-96F037), D-68838 (Asta Medica), D-68836 (Asta Medica), Myoseverin B, D-43411 (Zentaris, also known as D-81862), A-289099 (Abbott), A-318315 (Abbott), HTI-286 (also known as SPA-110, trifluoroacetate salt) (Wyeth), D-82317 (Zentaris), D-82318 (Zentaris), SC-12983 (NCI), Resverastatin phosphate sodium, BPR-OY-007 (National Health Research Institutes), and SSR-250411 (Sanofi).

Where the individual is suffering from or at risk of suffering from an autoimmune disease, an inflammatory disease, or an allergy disease, Compound 1 can be used in with one or more of the following therapeutic agents in any combination: immunosuppressants (e.g., tacrolimus, cyclosporin, rapamycin, methotrexate, cyclophosphamide, azathioprine, mercaptopurine, mycophenolate, or FTY720), glucocorticoids (e.g., prednisone, cortisone acetate, prednisolone, methylprednisolone, dexamethasone, betamethasone, triamcinolone, beclometasone, fludrocortisone acetate, deoxycorticosterone acetate, aldosterone), non-steroidal anti-inflammatory drugs (e.g., salicylates, arylalkanoic acids, 2-arylpropionic acids, N-arylanthranilic acids, oxicams, coxibs, or sulphonanilides), Cox-2-specific inhibitors (e.g., valdecoxib, celecoxib, or rofecoxib), leflunomide, gold thioglucose, gold thiomalate, aurofin, sulfasalazine, hydroxychloroquinine, minocycline, TNF-α binding proteins (e.g., infliximab, etanercept, or adalimumab), abatacept, anakinra, interferon-β, interferon-y, interleukin-2, allergy vaccines, antihistamines, antileukotrienes, beta-agonists, theophylline, or anticholinergics.

### Kits/Articles of Manufacture

For use in the therapeutic methods of use described herein, kits and articles of manufacture are also described herein. Such kits include a carrier, package, or container that is compartmentalized to receive one or more containers such as vials, tubes, and the like, each of the container(s) comprising one of the separate elements to be used in a method described herein. Suitable containers include, for example, bottles, vials, syringes, and test tubes. In one embodiment, the containers are formed from a variety of materials such as glass or plastic.

The articles of manufacture provided herein contain packaging materials. Packaging materials for use in packaging pharmaceutical products include, e.g., U.S. Patent Nos. 5,323,907. Examples of pharmaceutical packaging materials include, but are not limited to, blister packs, bottles, tubes, bags, containers, bottles, and any packaging material suitable for a selected formulation and intended mode of administration and treatment.

In some embodiments, the compounds or compositions described herein, are presented in a package or dispenser device which may contain one or more unit dosage forms containing the active ingredient. The compound or composition described herein is packaged alone, or packaged with another compound or another ingredient or additive. In some embodiments, the package contains one or more containers filled with one or more of the ingredients of the pharmaceutical compositions. In some embodiments, the package comprises metal or plastic foil, such as a blister pack. In some embodiments, the package or dispenser device is accompanied by instructions for administration, such as instructions for administering the compounds or compositions for treating a neoplastic disease. In some embodiments, the package or dispenser is accompanied with a notice associated with the container in form prescribed by a governmental agency regulating the manufacture, use, or sale of pharmaceuticals, which notice is reflective of approval by the agency of the form of the drug for human or veterinary administration. In some embodiments, such notice, for example, is the labeling approved by the U.S. Food and Drug Administration for prescription drugs, or the approved product insert. In some embodiments, compositions include a compound described herein formulated in a compatible pharmaceutical carrier are prepared, placed in an appropriate container, and labeled for treatment of an indicated condition. For example, the container(s) include Compound 1, optionally in a composition or in combination with another agent as disclosed herein. Such kits optionally include an identifying description or label or instructions relating to its use in the methods described herein.

A kit typically includes labels listing contents and/or instructions for use, and package inserts with instructions for use. A set of instructions will also typically be included.

In one embodiment, a label is on or associated with the container. In one embodiment, a label is on a container when letters, numbers or other characters forming the label are attached, molded or etched into the container itself; a label is associated with a container when it is present within a receptacle or carrier that also holds the container, e.g., as a package insert. In one embodiment, a label is used to indicate that the contents are to be used for a specific therapeutic application. The label also indicates directions for use of the contents, such as in the methods described herein.

In certain embodiments, the pharmaceutical compositions are presented in a pack or dispenser device which contains one or more unit dosage forms containing a compound provided herein. The pack, for example, contains metal or plastic foil, such as a blister pack. In one embodiment, the pack or dispenser device is accompanied by instructions for administration. In one embodiment, the pack or dispenser is also accompanied with a notice associated with the container in form prescribed by a governmental agency regulating the manufacture, use, or sale of pharmaceuticals, which notice is reflective of approval by the agency of the form of the drug for human or veterinary administration. Such notice, for example, is the labeling approved by the U.S. Food and Drug Administration for prescription drugs, or the approved product insert. In one embodiment, compositions containing a compound provided herein formulated in a compatible pharmaceutical carrier are also prepared, placed in an appropriate container, and labeled for treatment of an indicated condition.

### Examples

The following examples are intended to illustrate the present invention and should not be construed as a limitation of the scope of the present invention.

### Experimental Section

### Example 1

An example of such a composition may be described as follows in Tables 1 and 2, where certain compositions of the invention were prepared.

**Table 1: Qualitative and Quantitative composition of ibrutinib film coated tablets 560/140 mg**

| **Ingredient** | **Quality Reference** | **Function** | **560 (mg)** | | **140 (mg)** | |
|---|---|---|---|---|---|---|
| | | | **"A"** | | **"B"** | |
| | | | **mg/tab** | **%w/w** | **mg/tab** | **% w/w** |
| **Intragranular** | | | | | | |
| ibrutinib^{a} | Company Standard | Active | 560.0 | 67.96 | 140.000 | 67.96 |
| Silicified Microcrystalline Cellulose^{a} | NF | Filler | 93.28 | 11.32 | 23.32 | 11.32 |
| Sodium lauryl Sulfate | Ph.Eur. | Wetting agent | 37.6 | 4.56 | 9.400 | 4.56 |
| Crospovidone | Ph.Eur. | Disintegrant | 40.0 | 4.85 | 10.00 | 4.85 |
| Magnesium Stearate^{b} | Ph.Eur. | Lubricant | 4.00 | 0.49 | 1.00 | 0.49 |

| **Extragranular** | | | | | | |
|---|---|---|---|---|---|---|
| Sodium lauryl Sulfate | Ph.Eur. | Wetting agent | 18.4 | 2.23 | 4.600 | 2.23 |
| Crospovidone | Ph.Eur. | Disintegrant | 40.0 | 4.85 | 10.00 | 4.85 |
| Colloidal Silicon Dioxide | Ph.Eur. | Glidant | 4.0 | 0.49 | 1.00 | 0.49 |
| Magnesium Stearate^{b} | Ph.Eur. | Lubricant | 2.72 | 0.33 | 0.68 | 0.33 |
| **Total uncoated tablet weight (mg)** | | | **800** | | **200** | |
| Opadry (White) | Company Standard | Film coating agent | 24.00 | 2.91 | 6.00 | 2.91 |
| Purified Water^{c} | Company Standard | Vehicle | q.s. | | q.s. | |
| **Total coated tablet weight** | | | **824.00** | | **206.0** | |

| | | | | | | |
|---|---|---|---|---|---|---|
| ^{a} Quantity to be adjusted based on purity of ibrutinib ^{b} Non-bovine grade ^{c} Does not remain in finished product except in traces | | | | | | |

**Table 2: Qualitative and Quantitative composition of ibrutinib film coated tablets 560/140 mg**

| **Ingredient** | **Quality Reference** | **Function** | **560 (mg)** | | **140 (mg)** | |
|---|---|---|---|---|---|---|
| | | | **"C"** | | **"D"** | |
| | | | **mg/tab** | **%w/w** | **mg/tab** | **% w/w** |
| **Intragranular** | | | | | | |
| ibrutinib^{a} | Company Standard | Active | 560.0 | 57.84 | 140.000 | 57.84 |
| Silicified Microcrystalline Cellulose" | NF | Filler | 218.9 | 22.61 | 54.725 | 22.61 |
| Sodium lauryl Sulfate | Ph.Eur. | Wetting agent | 37.6 | 3.88 | 9.400 | 3.88 |
| Crospovidone | Ph.Eur. | Disintegrant | 47.0 | 4.85 | 11.750 | 4.85 |
| Magnesium Stearate^{b} | Ph.Eur. | Lubricant | 3.2 | 0.33 | 0.800 | 0.33 |

| **Extragranular** | | | | | | |
|---|---|---|---|---|---|---|
| Sodium lauryl Sulfate | Ph.Eur. | Wetting agent | 18.4 | 1.90 | 4.600 | 1.90 |
| Crospovidone | Ph.Eur. | Disintegrant | 47.0 | 4.85 | 11.750 | 4.85 |
| Colloidal Silicon Dioxide | Ph.Eur. | Glidant | 4.7 | 0.49 | 1.175 | 0.49 |
| Magnesium Stearate^{b} | Ph.Eur. | Lubricant | 3.2 | 0.33 | 0.800 | 0.33 |
| **Total uncoated tablet weight (mg)** | | | **940** | | **235** | |
| Opadry (White) | Company Standard | Film coating agent | 28.2 | 2.91 | 7.050 | 2.91 |
| Purified Water^{c} | Company Standard | Vehicle | q.s. | | q.s. | |
| **Total coated tablet weight** | | | **968.2** | | **242.05** | |

| | | | | | | |
|---|---|---|---|---|---|---|
| ^{a} Quantity to be adjusted based on purity of ibrutinib ^{b} Non-bovine grade ^{c} Does not remain in finished product except in traces | | | | | | |

### Example 2

Table 3 below also shows examples of the dry granulation "roller compaction" formulations (see the four "RC" formulations) that were prepared (where "PCI-32765 refers to "Ibrutinib" or a compound 1):

**Table 3**

| | RC, 60% API, API : SLS, 10:1. Crospovidone | | | | RC, 70% API, API : SLS, 10:1. Crospovidone | | | |
|---|---|---|---|---|---|---|---|---|
| Batch | Development Batch A | | Scale Up Batch A | | Development Batch B | | Scale up Batch B | |
| | **mg/tab** | **% w/w** | **mg/tab** | **% w/w** | **mg/tab** | **% w/w** | **mg/tab** | **% w/w** |
| **Intragranular layer** | | | | | | | | |
| PCI-32765 ("ibrutinib") | 560.0 | 60.0 | 560.0 | 59.6 | 560.0 | 69.9 | 560.0 | 70.0 |
| Silicified Microcrystalline Cellulose(HD90) | 212.9 | 22.8 | 218.9 | 23.3 | 94.7 | 11.8 | 93.3 | 11.7 |
| SLS (Kolliphor Fine) | 37.6 | 4.0 | 37.6 | 4.0 | 37.6 | 4.7 | 37.6 | 4.7 |
| Crospovidone XL | 46.7 | 5.0 | 47.0 | 5.0 | 40.0 | 5.0 | 40.0 | 5.0 |
| Magnesium Stearate | 3.2 | 0.3 | 3.2 | 0.3 | 4.0 | 0.5 | 4.0 | 0.5 |

| **Extragranular layer** | | | | | | | | |
|---|---|---|---|---|---|---|---|---|
| SLS (Kolliphor Fine) | 18.4 | 2.0 | 18.4 | 2.0 | 18.4 | 2.3 | 18.4 | 2.3 |
| Crospovidone XL | 46.7 | 5.0 | 47.0 | 5.0 | 40.0 | 5.0 | 40.0 | 5.0 |
| Silicon Dioxide (Aerosil 200) | 4.7 | 0.5 | 4.7 | 0.5 | 4.0 | 0.5 | 4.0 | 0.5 |
| Magnesium Stearate | 3.2 | 0.3 | 3.2 | 0.3 | 2.7 | 0.3 | 2.7 | 0.3 |
| **Total** | **933.3** | 100.0 | **940.0** | 100.0 | **801.4** | 100.0 | **800.0** | 100.0 |

In the above compositions, mannitol is not employed. However, as described herein, in an aspect, a water-soluble filler (such as a polyol, e.g. mannitol) may be a component of the pharmaceutical composition. Further, a film-coating may be employed as employed in Example 1, e.g. a film-coating agent such as opadry and purified water as the vehicle. In this case, the w/w percentages may change accordingly. Scale-up Batch A in Table 3 above is film-coated as described in Example 1 formulation "C", and is referred to herein as "Treatment C".

### Formulation Dimensions

As indicated herein, the tablet formulations of the invention were prepared having the following dimensions as indicated in Table 4 (but where the last column indicates the dimensions of the capsule that is already the subject of the FDA approval; the "thickness" of the capsule being uniform and hence no width is specified):

**Table 4**

| | 140 mg | 280 mg | 420 mg | 560 mg | 700 mg | 840 mg | "Treatment A" (140 mg capsule) |
|---|---|---|---|---|---|---|---|
| Length (mm) | | 15.1 | 17.7 | 19.2 | 21.1 | 22.1 | 21.7 |
| Width (mm) | 9.1 | 7.1 | 7.6 | 8.3 | 10.6 | 10.6 | |
| Thickness (mm) | 4.4 | 5.5 | 6.7 | 7.4 | 7.3 | 7.4 | 7.6 |
| Core weight (mg) | 235 | 470 | 705 | 940 | 1175 | 1410 | 330-333 (the fill) |
| Coated (mg) | 242 | 484 | 726 | 968 | 1210 | 1452 | 425 |

For the 140 mg dose tablet formulation described above, the length is not specified as it is substantially similar to the width and the tablet shape is therefore substantially a circle. For remaining formulations, where length, width and thickness is specified, the tablet shape is substantially an oblong (or an elongated rectangle).

### Example - Pharmaceutical Formulation/Process for Preparing

An example for a process for manufacturing such a pharmaceutical formulation may be described as follows:
1. Blend sieved sodium lauryl sulfate, silicified microcrystalline cellulose and Crospovidone in a blender
2. Blend with sieved one half of the amount of micronized ibrutinib
3. Blend with sieved, the remaining half of micronized ibrutinib.
4. The blended mass is lubricated with sieved magnesium stearate in a blender.
5. Compact the blend (intraganular) through roller compactor.
6. Pass compacted material through mill
7. Blend milled material with the second portion of sieved Crospovidone and sodium lauryl sulfate along with colloidal silicon dioxide in a blender.
8. The blended granulation is lubricated with the second portion of sieved magnesium stearate in a blender.
9. Final blend is compressed into tablets using rotary compression machine fitted with suitable tooling
10. Tablets are film coated using coating machine
11. Package tablets using conventional procedure.

The above process may also be adapted/amended depending on the components included in the pharmaceutical composition (e.g. SMCC HD90 as the microcrystalline cellulose employed).

### Biological Example

### A Single-Dose, Open-Label, Randomized, Crossover Study to Assess the Pharmacokinetics of Ibrutinib Tablet Formulations in Healthy Adult Subjects Compared to the Ibrutinib Capsule "Treatment A"

This is a single-centre, open-label, randomized, crossover, single-dose study in healthy adults. After providing written informed consent, subjects were screened within 21 days (Day -21 to -2).

**Main Criteria for Inclusion:** Healthy men and women between 18 and 55 years of age, inclusive; body mass index (BMI) between 18 and 30 kg/m², inclusive, and a body weight of not less than 50 kg. Women must be post-menopausal or surgically sterile.

Eligible subjects received a single oral dose of ibrutinib 560 mg either:
- as four capsules comprising 140 mg ibrutinib per capsule (see below for the capsule formulation), referred to hereinafter/in the Figures as "Treatment A"; or
- as a tablet formulation comprising 560 mg ibrutinib per tablet (as per a formulation of the invention, specifically "Scale-Up Batch A", which is referred to hereinafter and in the Figures as "Treatment C")
with 240 mL of noncarbonated water on Day 1 of each treatment period after fasting at least 10 hours before each dose. Water was allowed ad libitum beginning 2 hours after each dose, and lunch was provided beginning 4 hours after each dose.

### Ibrutinib capsule 140 mg (four capsules = "Treatment A")

This capsule manufacturing process includes the following steps: weigh the indicated amount of the components, mix together and add into an appropriate size capsule, and close capsule:

| Formulation - capsule | **140mg capsule** |
|---|---|
| Process | -- |
| Component | w/w % |
| Ibrutinib | 42.0 |
| Lactose Monohydrate NF | 0 |
| Microcrystalline cellulose NF | 46.5 |
| Hydroxypropyl Cellulose NF | 0 |
| Croscarmellose sodium NF | 7.0 |
| Sodium lauryl sulfate NF | 4.0 |
| Colloidal Silicon Dioxide NF | 0 |
| Magnesium stearate NF | 0.5 |
| Tablet Weight | 333.3 |

The capsules are stored at room temperature until they are used. Such capsules are those that are approved by the US FDA.

For the purposes of the Figures "Treatment B" is a different formulation not discussed/disclosed in this case, and may be ignored. "Treatment C" is a specific formulation of the invention, as described above.

Blood samples for pharmacokinetic (PK) analysis of ibrutinib were collected before dosing and over 48 hours after dosing in each treatment period.

Total duration of the study was approximately 70 days (21-day screening period, 4 x 3-day treatment periods with 7-day washouts between periods, and a 7-day follow-up phase).

PK parameters including the following were calculated and the following abbreviations used:
- Cₘₐₓ:: Maximum observed concentration
- Tₘₐₓ:: Time to reach the maximum observed concentration
- AUCₗₐₛₜ:: Area under the concentration-time curve from time 0 to last time point
- AUC_{∞}:: Area under the concentration-time curve from time 0 to infinite time
- t_{1/2}:: Apparent elimination half-life associated with the terminal slope of the semilogarithmic drug concentration-time curve

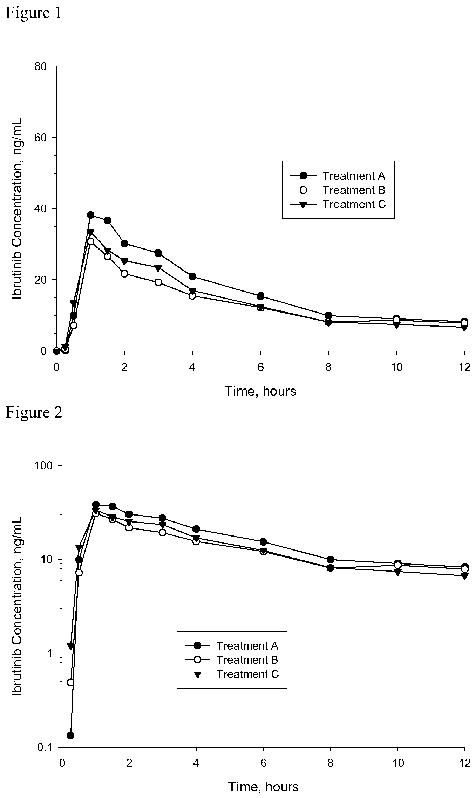

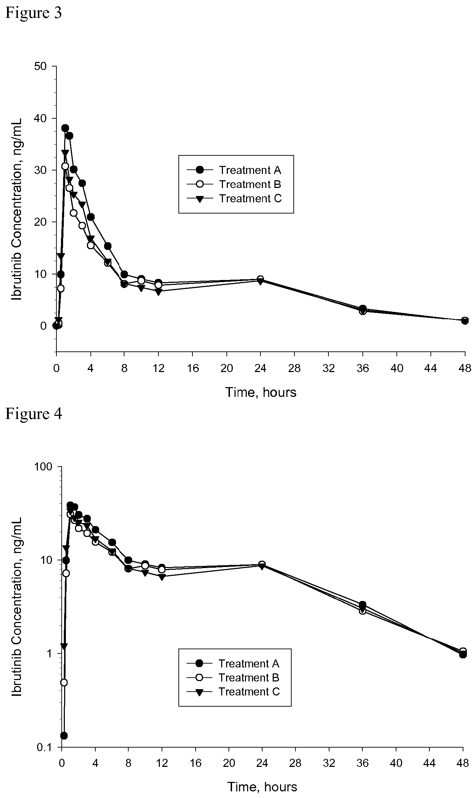

In some embodiments, the high load tablet formulations possess both pharmaceutically acceptable properties and desired PK properties, such as a high Cₘₐₓ, similar to that of a capsule formulation. This is exemplified in the Figures 1, 2, 3 and 4, where a comparison can be seen between "Treatment A" (n = 32; where n is the number of adult subjects) and "Treatment C" (n = 22).

Based on the above-mentioned results, the relative bioavailability of the tablet formulations of the invention may be calculated - exposure compared to the reference capsule formulation may be measured. For instance in the following table:

### Pharmacokinetics Parameters and Results

| **Treatment** | **n** | | **Cₘₐₓ (ng/mL)** | **Tₘₐₓ (h) - median** | **AUCₗₐₛₜ (ng*h/mL)** | **AUC_{inf} (ng*h/mL)** | **T_{1/2} (h)** |
|---|---|---|---|---|---|---|---|
| **Treatment A** | 32 | Mean | 48.6 | 1.00 | 379 | a 465 | a 9.5 |
| Ibrutinib capsule Formulation A (4 x 140 mg/capsule) | | | | | | | |
| | | SD | 36.0 | | 248 | 248 | 3.5 |
| **Treatment C** | 22 | Mean | 39.2 | 1.26 | 351 | 399^{c} | 9.2^{c} |
| Ibrutinib Tablet Formulation (dry granulation RC and coated) - 560 mg/tablet | | | | | | | |
| | | SD | 20.4 | | 158 | 171 | 2.3 |

| | | | | | | | |
|---|---|---|---|---|---|---|---|
| ^{a}n = 22; ^{b} n = 12; ^{c} n = 13 (and n = number of adult subjects) | | | | | | | |

In the table above, there is an exception in the "Mean" rows provided, where "Tₘₐₓ (h)" is the median value. For Treatment B and C, the intrasubject variability was relatively high for Cₘₐₓ but moderate for AUCₗₐₛₜ and AUC_{inf} (or AUC_{∞}).

Based on the above results, it can be seen that:
- half-life for Treatment C was similar to that of Treatment A
- relative bioavailability of Treatment C could compare favourably to Treatment A
- Treatment C could provide similar exposure to Treatment A

Based on the above results, in an aspect, there is provided a formulation in which:
- the GMR (geometric mean ratio) ranges from 75% to 92% (e.g. 80 to 85%) for Cₘₐₓ;
- the GMR for AUCₗₐₛₜ ranges from 85% to 110% (e.g. from 85 to 100%, or 85 to 95%); and/or
- the GMR for AUC_{inf} (or AUC_{∞}) ranges from 80% to 105% (e.g. from 85 to 95%).

Such features relating to exposure may be a part of any of the embodiments disclosed herein.

Ibrutinib was well tolerated after the single-dose administration (using the particular formulation of the invention mentioned above "Treatment C") in healthy subjects. No new or unanticipated safety signals were identified.

### Further Biological Examples

Studies are performed to test the safety, tolerability and/or efficacy of the formulations of the invention (particularly the high-load 560 mg formulation) in subjects with a disease as defined herein (e.g. chronic lymphocytic leukemia, relapsed/refractory mantle cell lymphoma, etc). Similar studies may also be performed to test such formulations in combination (as described herein).

## Claims

1. A high-load solid tablet formulation comprising a pharmaceutical composition comprising ibrutinib, wherein ibrutinib is a compound with the structure of Compound 1, 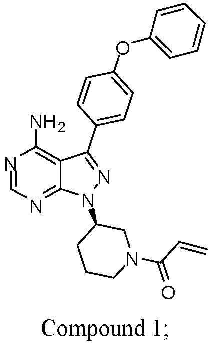
and wherein the pharmaceutical composition comprises i) 50% w/w to 80% w/w of ibrutinib, ii) excipients comprising:
- 10-30% w/w of filler that is microcrystalline cellulose of the total weight of the pharmaceutical composition;
- 5-20% w/w of disintegrant of the total weight of the pharmaceutical composition, and one or more additional pharmaceutically acceptable excipients,
and further wherein
- the pharmaceutical composition comprises intragranular and extragranular ingredients
- the pharmaceutical composition is prepared using a dry granulation method

2. The high-load solid tablet formulation of claim 1, wherein the disintegrant is crospovidone.

3. The high-load solid tablet formulation of claim 1 or claim 2, wherein the excipients do not comprise a filler that is mannitol or wherein the microcrystalline cellulose is the only filler as a component of the pharmaceutical composition.

4. The high-load solid tablet formulation of any of the preceding claims, wherein the pharmaceutical composition comprises 60% w/w to 80% w/w of ibrutinib, 60% w/w to 70% w/w of ibrutinib, or 60% or 70% w/w of ibrutinib.

5. The high-load solid tablet formulation of any one of claims 1-4, wherein the microcrystalline cellulose is silicified microcrystalline cellulose.

6. The high-load solid tablet formulation of any one of claims 1-5, wherein ibrutinib and the microcrystalline cellulose are intragranular ingredients.

7. The high-load solid tablet formulation of any one of claims 1-6, wherein the pharmaceutical composition comprises 10% w/w to about 25% w/w of the microcrystalline cellulose, about 22-23% w/w of the microcrystalline cellulose, or about 12% w/w of the microcrystalline cellulose, wherein "about" used before a numerical value indicates that the value may vary within ± 10% of the stated value.

8. The high-load solid tablet formulation of any one of claims 1-7, wherein crospovidone is an intragranular and extragranular ingredient.

9. The high-load solid tablet formulation of any one of claims 1-8, wherein the pharmaceutical composition comprises 8% w/w to 12% w/w of crospovidone, or 10% w/w of crospovidone.

10. The high-load solid tablet formulation of any of claims 1-9, wherein the pharmaceutical composition comprises (i) 60% w/w of ibrutinib, 22-23% w/w of microcrystalline cellulose, and 10% w/w of crospovidone; or (ii) 70% w/w of ibrutinib, 12% w/w of microcrystalline cellulose, and 10% w/w of crospovidone.

11. The high-load solid tablet formulation of any one of claims 1-10, wherein the pharmaceutical composition is prepared using a dry granulation method that is a roller compaction process.

12. The high-load solid tablet formulation of any of claims 1-11, wherein the one or more additional excipients are present in an amount from 7% w/w to 13% w/w.

13. The high-load solid tablet formulation of any one claims 1-12, wherein the one or more additional excipients are selected from the group consisting of binders, lubricants, glidants, and surfactants.

14. The high-load solid tablet formulation of any one claims 1-13, wherein at least one additional excipient is a surfactant.

15. The high-load solid tablet formulation of claim 14, wherein the surfactant is sodium lauryl sulfate.

16. The high-load solid tablet formulation of claim 15, wherein the sodium lauryl sulfate is present in an amount from 0 to 10% w/w, 4% w/w to 8% w/w, or 4% w/w to 6% w/w.

17. The high-load solid tablet formulation of any one claims 1-16, wherein at least one additional excipient is a glidant.

18. The high-load solid tablet formulation of claim 17, wherein the glidant is silica (colloidal silicon dioxide).

19. The high-load solid tablet formulation of claim 18, wherein the silica (colloidal silicon dioxide) is present in an amount from 0 to 5% w/w, 0.1% w/w to 1.5% w/w, 0.4% w/w to 0.8% w/w, or 0.5% w/w).

20. The high-load solid tablet formulation of any one claims 1-19, wherein at least one additional excipient is a lubricant.

21. The high-load solid tablet formulation of claim 20, wherein the lubricant is magnesium stearate.

22. The high-load solid tablet formulation of claim 21, wherein the magnesium stearate is present in an amount from 0.01% w/w to 5% w/w, 0.01% w/w to 2% w/w, 0.1% w/w to 0.7% w/w, or 0.3% w/w to 0.5% w/w.

23. The high-load solid tablet formulation of any one of claims 1-22,
wherein a binder is not present in the formulation (as an excipient).

24. The high-load solid tablet formulation of any one of claims 1-23,
wherein the total weight of a tablet is 800 mg.

25. The high-load solid tablet formulation of any one of claims 1-24,
wherein ibrutinib is in an amount of 560 mg.

26. The high-load solid tablet formulation of any one of claims 1-25,
wherein ibrutinib is in micronized form.

27. The high-load solid tablet formulation of any one of claims 1-26,
wherein the formulation is used for once a day dosing.

28. The high-load solid tablet formulation of any one of claims 1-27,
wherein the formulation is in an oral dosage form.

29. A tablet formulation of any one of claims 1-28 for use as a medicament.

30. A tablet formulation of any one of claims 1-28 for use in the treatment of: an autoimmune disease; a heteroimmune disease; a cancer; mastocytosis; osteoporosis or bone resorption disorders; an inflammatory disease or condition; lupus; and/or a heteroimmune disease or condition.

31. The tablet formulation for use as claimed in claim 30, wherein the autoimmune disease is rheumatoid arthritis or lupus.

32. The tablet formulation for use as claimed in claim 30, wherein the cancer is: a B-cell proliferative disorder; a diffuse large B cell lymphoma, follicular lymphoma or chronic lymphocytic leukemia; a cancer that is a a B cell malignancy; a cancer that is a B cell malignancy selected from chronic lymphocytic leukemia (CLL)/ small lymphocytic lymphoma (SLL), mantle cell lymphoma (MCL), diffuse large B Cell lymphoma (DLBCL), and multiple myeloma; a cancer that is a lymphoma, leukemia or a solid tumor; or a diffuse large B cell lymphoma, follicular lymphoma, chronic lymphocytic lymphoma, chronic lymphocytic leukemia, B-cell prolymphocytic leukemia, lymphoplasmacytic lymphoma/Waldenström macroglobulinemia, splenic marginal zone lymphoma, plasma cell myeloma, plasmacytoma, extranodal marginal zone B cell lymphoma, nodal marginal zone B cell lymphoma, mantle cell lymphoma, mediastinal (thymic) large B cell lymphoma, intravascular large B cell lymphoma, primary effusion lymphoma, burkitt lymphomalleukemia, or lymphomatoid granulomatosis.

33. A process for preparing the tablet formulation of any one of claims 1-28, the process comprising preparing dry granules comprising ibrutinib and at least one excipient by a dry granulation method.

34. A process as claimed in claim 33, wherein the dry granulation method is a roller granulation method.

## Patentansprüche

1. Hochbeladene feste Tablettenformulierung, umfassend eine pharmazeutische Zusammensetzung, die Ibrutinib umfasst, wobei Ibrutinib eine Verbindung mit der Struktur von Verbindung 1 ist, 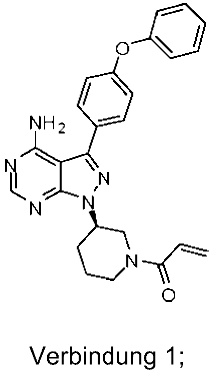
und wobei die pharmazeutische Zusammensetzung i) 50 Gew.-% bis 80 Gew.- % Ibrutinib, ii) Hilfsstoffe umfasst, umfassend:
- 10 bis 30 Gew.-% Füllstoff in Form von mikrokristalliner Zellulose vom Gesamtgewicht der pharmazeutischen Zusammensetzung;
- 5 bis 20 Gew.-% Sprengmittel vom Gesamtgewicht der pharmazeutischen Zusammensetzung und ein oder mehrere zusätzliche pharmazeutisch verträgliche Hilfsstoffe,
und wobei ferner
- die pharmazeutische Zusammensetzung intragranuläre und extragranuläre Inhaltsstoffe umfasst
- die pharmazeutische Zusammensetzung unter Verwendung eines Trockengranulationsverfahrens hergestellt wird.

2. Hochbeladene feste Tablettenformulierung nach Anspruch 1, wobei das Sprengmittel Crospovidon ist.

3. Hochbeladene feste Tablettenformulierung nach Anspruch 1 oder Anspruch 2, wobei die Hilfsstoffe keinen Füllstoff umfassen, der Mannitol ist, oder wobei die mikrokristalline Cellulose der einzige Füllstoff als Bestandteil der pharmazeutischen Zusammensetzung ist.

4. Hochbeladene feste Tablettenformulierung nach einem der vorstehenden Ansprüche, wobei die pharmazeutische Zusammensetzung 60 Gew.-% bis 80 Gew.-% Ibrutinib, 60 Gew.-% bis 70 Gew.-% Ibrutinib oder 60 Gew.-% oder 70 Gew.-% Ibrutinib umfasst.

5. Hochbeladene feste Tablettenformulierung nach einem der Ansprüche 1 bis 4, wobei die mikrokristalline Cellulose verkieselte mikrokristalline Cellulose ist.

6. Hochbeladene feste Tablettenformulierung nach einem der Ansprüche 1 bis 5, wobei Ibrutinib und die mikrokristalline Cellulose intragranuläre Inhaltsstoffe sind.

7. Hochbeladene feste Tablettenformulierung nach einem der Ansprüche 1 bis 6, wobei die pharmazeutische Zusammensetzung 10 Gew.-% bis etwa 25 Gew.-% mikrokristalline Cellulose, etwa 22 bis 23 Gew.-% mikrokristalline Cellulose oder etwa 12 Gew.-% mikrokristalline Cellulose umfasst, wobei die Verwendung von "etwa" vor einem numerischen Wert angibt, dass der Wert innerhalb von ± 10 % des angegebenen Werts variieren kann.

8. Hochbeladene feste Tablettenformulierung nach einem der Ansprüche 1 bis 7, wobei Crospovidon ein intragranulärer und extragranulärer Inhaltsstoff ist.

9. Hochbeladene feste Tablettenformulierung nach einem der Ansprüche 1 bis 8, wobei die pharmazeutische Zusammensetzung 8 Gew.-% bis 12 Gew.-% Crospovidon oder 10 Gew.-% Crospovidon umfasst.

10. Hochbeladene feste Tablettenformulierung nach einem der Ansprüche 1 bis 9, wobei die pharmazeutische Zusammensetzung (i) 60 Gew.-% Ibrutinib, 22 bis 23 Gew.-% mikrokristalline Cellulose und 10 Gew.-% Crospovidon umfasst; oder (ii) 70 Gew.-% Ibrutinib, 12 Gew.-% mikrokristalline Cellulose und 10 Gew.-% Crospovidon umfasst.

11. Hochbeladene feste Tablettenformulierung nach einem der Ansprüche 1 bis 10, wobei die pharmazeutische Zusammensetzung unter Verwendung eines Trockengranulationsverfahrens hergestellt wird, bei dem es sich um ein Walzenkompaktierungsverfahren handelt.

12. Hochbeladene feste Tablettenformulierung nach einem der Ansprüche 1 bis 11, wobei der eine oder die mehreren zusätzlichen Hilfsstoffe in einer Menge von 7 Gew.-% bis 13 Gew.-% vorhanden sind.

13. Hochbeladene feste Tablettenformulierung nach einem der Ansprüche 1 bis 12, wobei der eine oder die mehreren zusätzlichen Hilfsstoffe ausgewählt sind aus der Gruppe bestehend aus Bindemitteln, Schmiermitteln, Gleitmitteln und Tensiden.

14. Hochbeladene feste Tablettenformulierung nach einem der Ansprüche 1 bis 13, wobei mindestens ein zusätzlicher Hilfsstoff ein Tensid ist.

15. Hochbeladene feste Tablettenformulierung nach Anspruch 14, wobei das Tensid Natriumlaurylsulfat ist.

16. Hochbeladene feste Tablettenformulierung nach Anspruch 15, wobei das Natriumlaurylsulfat in einer Menge von 0 bis 10 Gew.-%, 4 Gew.-% bis 8 Gew.-% oder 4 Gew.-% bis 6 Gew.-% vorhanden ist.

17. Hochbeladene feste Tablettenformulierung nach einem der Ansprüche 1 bis 16, wobei mindestens ein zusätzlicher Hilfsstoff ein Gleitmittel ist.

18. Hochbeladene feste Tablettenformulierung nach Anspruch 17, wobei das Gleitmittel Kieselerde (kolloidales Siliciumdioxid) ist.

19. Hochbeladene feste Tablettenformulierung nach Anspruch 18, wobei die Kieselerde (kolloidales Siliciumdioxid) in einer Menge von 0 bis 5 Gew.-%, 0,1 Gew.-% bis 1,5 Gew.-%, 0,4 Gew.-% bis 0,8 Gew.-% oder von 0,5 Gew.-% vorhanden ist.

20. Hochbeladene feste Tablettenformulierung nach einem der Ansprüche 1 bis 19, wobei mindestens ein zusätzlicher Hilfsstoff ein Schmiermittel ist.

21. Hochbeladene feste Tablettenformulierung nach Anspruch 20, wobei das Schmiermittel Magnesiumstearat ist.

22. Hochbeladene feste Tablettenformulierung nach Anspruch 21, wobei das Magnesiumstearat in einer Menge von 0,01 Gew.-% bis 5 Gew.-%, 0,01 Gew.-% bis 2 Gew.-%, 0,1 Gew.-% bis 0,7 Gew.-% oder von 0,3 Gew.-% bis 0,5 Gew.-% vorhanden ist.

23. Hochbeladene feste Tablettenformulierung nach einem der Ansprüche 1 bis 22, wobei in der Formulierung kein Bindemittel (als Hilfsstoff) vorhanden ist.

24. Hochbeladene feste Tablettenformulierung nach einem der Ansprüche 1 bis 23, wobei das Gesamtgewicht einer Tablette 800 mg beträgt.

25. Hochbeladene feste Tablettenformulierung nach einem der Ansprüche 1 bis 24, wobei Ibrutinib in einer Menge von 560 mg vorliegt.

26. Hochbeladene feste Tablettenformulierung nach einem der Ansprüche 1 bis 25, wobei Ibrutinib in mikronisierter Form vorliegt.

27. Hochbeladene feste Tablettenformulierung nach einem der Ansprüche 1 bis 26, wobei die Formulierung für eine einmal tägliche Dosierung verwendet wird.

28. Hochbeladene feste Tablettenformulierung nach einem der Ansprüche 1 bis 27, wobei die Formulierung in einer oralen Dosierungsform vorliegt.

29. Tablettenformulierung nach einem der Ansprüche 1 bis 28 zur Verwendung als ein Arzneimittel.

30. Tablettenformulierung nach einem der Ansprüche 1 bis 28 zur Verwendung bei der Behandlung von: einer Autoimmunerkrankung; einer Heteroimmunerkrankung; einem Krebs; Mastozytose; Osteoporose oder Knochenresorptionsstörungen; einer entzündlichen Erkrankung oder entzündlichen Beschwerden; Lupus; und/oder einer Heteroimmunerkrankung oder einem heteroimmunen Zustand.

31. Tablettenformulierung zur Verwendung nach Anspruch 30, wobei die Autoimmunerkrankung rheumatoide Arthritis oder Lupus ist.

32. Tablettenformulierung zur Verwendung nach Anspruch 30, wobei der Krebs ist: eine B-Zell-proliferative Störung; ein diffuses großzelliges B-Zell-Lymphom, follikuläres Lymphom oder chronische lymphatische Leukämie; ein Krebs, der ein B-Zell-Malignom ist; ein Krebs, der ein B-Zell-Malignom ist, das aus chronischer lymphatischer Leukämie (CLL)/kleinzelligem lymphatischem Lymphom (SLL), Mantelzelllymphom (MCL), diffusem großzelligem B-Zell-Lymphom (DLBCL) und multiplem Myelom ausgewählt ist; ein Krebs, der ein Lymphom, eine Leukämie oder ein solider Tumor ist; oder ein diffuses großzelliges B-Zell-Lymphom, follikuläres Lymphom, chronisches lymphatisches Lymphom, chronische lymphatische Leukämie, B-Zell-Prolymphozytäre Leukämie, lymphoplasmazytisches Lymphom/Waldenström-Makroglobulinämie, splenisches Marginalzonenlymphom, Plasmazellmyelom, Plasmozytom, extranodales Marginalzonen-B-Zell-Lymphom, nodales Marginalzonen-B-Zell-Lymphom, Mantelzelllymphom, mediastinales (Thymus-)großzelliges B-Zell-Lymphom, intravaskuläres großzelliges B-Zell-Lymphom, primäres Effusionslymphom, Burkitt-Lymphom/Leukämie oder lymphomatoide Granulomatose.

33. Prozess zum Herstellen der Tablettenformulierung nach einem der Ansprüche 1 bis 28, wobei der Prozess das Herstellen von Trockengranalien, die Ibrutinib und mindestens einen Hilfsstoff umfassen, durch ein Trockengranulationsverfahren umfasst.

34. Verfahren nach Anspruch 33, wobei das Trockengranulationsverfahren ein Walzengranulationsverfahren ist.

## Revendications

1. Formulation de comprimé solide à forte charge comprenant une composition pharmaceutique comprenant de l'ibrutinib, dans laquelle l'ibrutinib est un composé avec la structure du Composé 1, 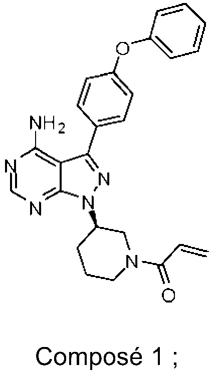
et dans laquelle la composition pharmaceutique comprend i) 50 % en poids à 80 % en poids d'ibrutinib, ii) des excipients comprenant :
- 10 à 30 % en poids de charge qui est de la cellulose microcristalline du poids total de la composition pharmaceutique ;
- 5 à 20 % en poids de délitant du poids total de la composition pharmaceutique, et un ou plusieurs excipients pharmaceutiquement acceptables supplémentaires,
et dans laquelle en outre
- la composition pharmaceutique comprend des ingrédients intragranulaires et extragranulaires
la composition pharmaceutique est préparée à l'aide d'un procédé de granulation à sec.

2. Formulation de comprimé solide à forte charge selon la revendication 1, dans laquelle le délitant est de la crospovidone.

3. Formulation de comprimé solide à forte charge selon la revendication 1 ou la revendication 2, dans laquelle les excipients ne comprennent pas de charge qui est du mannitol ou dans laquelle la cellulose microcristalline est la seule charge en tant que composant de la composition pharmaceutique.

4. Formulation de comprimé solide à forte charge selon l'une quelconque des revendications précédentes, dans laquelle la composition pharmaceutique comprend 60 % en poids à 80 % en poids d'ibrutinib, 60 % en poids à 70 % en poids d'ibrutinib, ou 60 % ou 70 % en poids d'ibrutinib.

5. Formulation de comprimé solide à forte charge selon l'une quelconque des revendications 1 à 4, dans laquelle la cellulose microcristalline est de la cellulose microcristalline silicifiée.

6. Formulation de comprimé solide à forte charge selon l'une quelconque des revendications 1 à 5, dans laquelle l'ibrutinib et la cellulose microcristalline sont des ingrédients intragranulaires.

7. Formulation de comprimé solide à forte charge selon l'une quelconque des revendications 1 à 6, dans laquelle la composition pharmaceutique comprend 10 % en poids à environ 25 % en poids de la cellulose microcristalline, environ 22 à 23 % en poids de la cellulose microcristalline, ou environ 12 % en poids de la cellulose microcristalline, dans laquelle « environ » utilisé avant une valeur numérique indique que la valeur peut varier à ±10 % de la valeur indiquée.

8. Formulation de comprimé solide à forte charge selon l'une quelconque des revendications 1 à 7, dans laquelle la crospovidone est un ingrédient intragranulaire et extragranulaire.

9. Formulation de comprimé solide à forte charge selon l'une quelconque des revendications 1 à 8, dans laquelle la composition pharmaceutique comprend 8 % en poids à 12 % en poids de crospovidone, ou 10 % en poids de crospovidone.

10. Formulation de comprimé solide à forte charge selon l'une quelconque des revendications 1 à 9, dans laquelle la composition pharmaceutique comprend (i) 60 % en poids d'ibrutinib, 22 à 23 % en poids de cellulose microcristalline, et 10 % en poids de crospovidone ; ou (ii) 70 % en poids d'ibrutinib, 12 % en poids de cellulose microcristalline, et 10 % en poids de crospovidone.

11. Formulation de comprimé solide à forte charge selon l'une quelconque des revendications 1 à 10, dans laquelle la composition pharmaceutique est préparée à l'aide d'un procédé de granulation à sec qui est un processus de compaction à rouleaux.

12. Formulation de comprimé solide à forte charge selon l'une quelconque des revendications 1 à 11, dans laquelle le ou les excipients supplémentaires sont présents en une quantité allant de 7 % en poids à 13 % en poids.

13. Formulation de comprimé solide à forte charge selon l'une quelconque des revendications 1 à 12, dans laquelle le ou les excipients supplémentaires sont choisis dans le groupe constitué de liants, lubrifiants, agents glissants et agents tensioactifs.

14. Formulation de comprimé solide à forte charge selon l'une quelconque des revendications 1 à 13, dans laquelle au moins un excipient supplémentaire est un agent tensioactif.

15. Formulation de comprimé solide à forte charge selon la revendication 14, dans laquelle l'agent tensioactif est du laurylsulfate de sodium.

16. Formulation de comprimé solide à forte charge selon la revendication 15, dans laquelle le laurylsulfate de sodium est présent en une quantité allant de 0 à 10 % en poids, 4 % en poids à 8 % en poids, ou 4 % en poids à 6 % en poids.

17. Formulation de comprimé solide à forte charge selon l'une quelconque des revendications 1 à 16, dans laquelle au moins un excipient supplémentaire est un agent glissant.

18. Formulation de comprimé solide à forte charge selon la revendication 17, dans laquelle l'agent glissant est de la silice (dioxyde de silicium colloïdal).

19. Formulation de comprimé solide à forte charge selon la revendication 18, dans laquelle la silice (dioxyde de silicium colloïdal) est présente en une quantité allant de 0 à 5 % en poids, 0,1 % en poids à 1,5 % en poids, 0,4 % en poids à 0,8 % en poids, ou 0,5 % en poids).

20. Formulation de comprimé solide à forte charge selon l'une quelconque des revendications 1 à 19, dans laquelle au moins un excipient supplémentaire est un lubrifiant.

21. Formulation de comprimé solide à forte charge selon la revendication 20, dans laquelle le lubrifiant est du stéarate de magnésium.

22. Formulation de comprimé solide à forte charge selon la revendication 21 dans laquelle le stéarate de magnésium est présent en une quantité allant de 0,01 % en poids à 5 % en poids, 0,01 % en poids à 2 % en poids, 0,1 % en poids à 0,7 % en poids, ou 0,3 % en poids à 0,5 % en poids.

23. Formulation de comprimé solide à forte charge selon l'une quelconque des revendications 1 à 22, dans laquelle un liant n'est pas présent dans la formulation (en tant qu'excipient).

24. Formulation de comprimé solide à forte charge selon l'une quelconque des revendications 1 à 23, dans laquelle le poids total d'un comprimé est de 800 mg.

25. Formulation de comprimé solide à forte charge selon l'une quelconque des revendications 1 à 24, dans laquelle l'ibrutinib est en une quantité de 560 mg.

26. Formulation de comprimé solide à forte charge selon l'une quelconque des revendications 1 à 25, dans laquelle l'ibrutinib est sous forme micronisée.

27. Formulation de comprimé solide à forte charge selon l'une quelconque des revendications 1 à 26, dans laquelle la formulation est utilisée pour une posologie d'une fois par jour.

28. Formulation de comprimé solide à forte charge selon l'une quelconque des revendications 1 à 27, dans laquelle la formulation est sous une forme pharmaceutique orale.

29. Formulation de comprimé selon l'une quelconque des revendications 1 à 28 pour une utilisation en tant que médicament.

30. Formulation de comprimé selon l'une quelconque des revendications 1 à 28 pour une utilisation dans le traitement de : une maladie auto-immune ; une maladie hétéro-immune ; un cancer ; une mastocytose ; une ostéoporose ou des troubles de résorption osseuse ; une maladie ou affection inflammatoire ; un lupus ; et/ou une maladie ou affection hétéro-immune.

31. Formulation de comprimé pour utilisation selon la revendication 30, dans laquelle la maladie auto-immune est une polyarthrite rhumatoïde ou un lupus.

32. Formulation de comprimé pour utilisation selon la revendication 30, dans laquelle le cancer est : un trouble prolifératif des cellules B ; un lymphome diffus à grandes cellules B, un lymphome folliculaire ou une leucémie lymphocytaire chronique ; un cancer qui est une malignité à cellules B ; un cancer qui est une malignité à cellules B choisie parmi leucémie lymphocytaire chronique (LLC) / lymphome lymphocytaire de petite taille (SLL), lymphome à cellules du manteau (MCL), lymphome diffus à grandes cellules B (DLBCL) et myélome multiple. ; un cancer qui est un lymphome, une leucémie ou une tumeur solide ; ou un lymphome diffus à grandes cellules B, un lymphome folliculaire, un lymphome lymphocytaire chronique, une leucémie lymphocytaire chronique, une leucémie prolymphocytaire à cellules B, un lymphome lymphoplasmocytaire/une macroglobulinémie de Waldenström, un lymphome de la zone marginale splénique, un myélome plasmocytaire, un plasmocytome, un lymphome à cellules B de la zone marginale extranodale, un lymphome à cellules B de la zone marginale ganglionnaire, un lymphome à cellules du manteau, un lymphome médiastinal (thymique) à grandes cellules B, un lymphome intravasculaire à grandes cellules B, un lymphome de l'épanchement primaire, un lymphome/leucémie de Burkitt ou une granulomatose lymphomatoïde.

33. Processus permettant de préparer la formulation de comprimé selon l'une quelconque des revendications 1 à 28, le processus comprenant la préparation de granules secs comprenant de l'ibrutinib et au moins un excipient par un procédé de granulation à sec.

34. Processus selon la revendication 33, dans lequel le procédé de granulation à sec est un procédé de granulation au rouleau.
